# EUROPEAN PATENT APPLICATION

(11) **EP 4 785 991 A1**
(43) Date of publication of application: **05.08.2026**
(21) Application number: 26153658.5
(22) Date of filing: 23.01.2026
(51) Int. Cl.: A61N 1/37, A61B 5/00, A61N 1/362

(54) **DENOISING BIOLOGICAL SIGNALS**

(30) Priority: 04.02.2025 US 202563753799 P; 21.01.2026 US 202619454807
(71) Applicant: Pacesetter, Inc., Sylmar, CA 91342 (US)
(72) Inventor: JIANG, Ruoli, Sylmar, CA 91342 (US); SISON, Shiloh, Sylmar, CA 91342 (US)
(74) Representative: Barker Brettell Sweden AB

(57) **Abstract**

Disclosed herein is an implantable medical device (IMD) (100), and system (5) for denoising biological signals. The IMD (100) generates a biological signal representative of a physiologic parameter of a patient (3) and normally analyzes the biological signal to detect a characteristic of interest (COI) indicative of a pathophysiologic condition of the patient (3). The present technology denoises, when the IMD (100) is exposed to a noise causing condition, noisy biological signal segments of the biological signal generated while the IMD (100) is exposed to the noise causing condition using a denoising prediction model (400) to obtain denoised biological signal segments. The denoising prediction model (400) is trained to predict a denoised biological signal segment from a noisy biological signal segment. The denoised biological signal segments can then be analyzed to detect the COI indicative of the pathophysiologic condition of the patient (3).

## Description

### TECHNICAL FIELD

The present technology generally relates to methods, implantable medical devices (IMDs) and systems comprising IMDs, and in particular to such methods, IMDs and systems for denoising biological signals sensed by the IMDs.

### BACKGROUND

Magnetic resonance imaging (MRI) is an effective non-invasive imaging technique for generating sharp images of the internal anatomy of the human body, which provides an efficient means for diagnosing disorders, such as neurological and cardiac abnormalities and for spotting tumors. Briefly, the patient is placed within the center of a large superconducting magnet of a magnetic resonance (MR) scanner that generates a powerful static magnetic field. The static magnetic field causes protons within tissues of the body to align with an axis of the static field. A pulsed radio-frequency (RF) magnetic field is then applied causing precession of the protons around the axis of the static field. Pulsed gradient magnetic fields are then applied to cause the protons within selected locations of the body to emit RF signals, which are detected by sensors of the MRI system. Based on the RF signals emitted by the protons, the MRI system then generates a precise image of the selected locations of the body, typically image slices of organs of interest.

A significant problem with MRI is that its strong magnetic fields can interfere with the operation of implantable medical devices (IMDs), such as a pacemaker, a cardiac resynchronization therapy (CRT) device, an implantable cardioverter defibrillator (ICD) device, a neurostimulation (NS) device, an implantable cardiac monitor (ICM), a leadless IMD, such as a leadless pacemaker (LP), and other electronic devices implanted within the patient. The IMD may include one or more electrodes located on the housing of the IMD and/or include one or more electrodes located on lead(s) that are coupled to the IMD. The IMD and/or lead, or portions thereof, may be configured to be implanted subcutaneously, within or on one or more cardiac chambers, transvenous proximate the spine, within the skull and/or the like. IMDs are configured to sense and collect various types of biological signals, analyze the biological signals for various conditions, in some cases communicate the biological signals and/or conditions to an external device, and/or deliver therapy. The therapy may include delivering pacing pulses, delivering defibrillation shocks, delivering neurostimulation pulses, releasing a drug, transmitting a notification and/or the like.

The leads, and/or the IMD itself, may also have a variety of sensors for sensing one or more physiological signals within tissues or organs of the patient, such as electrical sensors, pressure sensors, temperature sensors, SvO2 sensors, photoplethysmography (PPG) sensors and/or the like. The sensors are typically connected to the IMD via electrical signal conduction paths within the various leads and/or within a housing of the IMD so as to receive control signals from the IMD and to relay sensed signals back to the IMD.

With conventional IMDs, the strong MRI fields can prevent the IMD from reliably sensing signals from the various electrodes of the leads and from the various physiological sensors, resulting in improper and/or dangerous operation. Therefore, some MRI conditional implants are placed in an MRI mode and/or programmed with MRI settings that may temporarily limit, or disable, the IMD's normal device operation and intended therapy delivery. For example, a pacemaker may be programmed to a predetermined pacing setting and native/inherent signal detection is discontinued prior to and during the MRI session. After the MRI session is complete, the previous settings of the IMD have to be restored, and the proper operation of the IMD and health of the patient have to be verified. While the MRI settings of some IMDs can be set by the patient, many devices require personnel, such as IMD clinic staff and/or staff of the IMD manufacturer, to program the IMD into the MRI mode and then return the IMD to normal operation. In many situations, personnel with expertise in the IMD(s) need to be on-site at the MRI clinic to accomplish this task.

Furthermore, while the IMD is in the MRI mode, the sensing of biological signals or the analysis thereof is disabled as the sensed biological signals are regarded as being too noisy to be used by the IMD for analysis.

To address these concerns, certain procedures must be followed to ensure the safety of the patient during the MRI session as well as after the MRI session is completed. Currently, participants in the scheduling and scanning processes can include the patient, the referring physician/clinic staff, staff of the MRI clinic, staff of the implant clinic, and the implant manufacturer's staff (e.g., field staff or field representatives, technical support services), etc., all of whom coordinate care in-person, and through phone calls and fax. Accordingly, a significant burden is placed on all participants.

### SUMMARY

It is a general objective to enable analysis of biological signal segments also when an implantable medical device (IMD) is exposed to noise causing conditions. This and other objectives are met by embodiments of the present technology as disclosed herein.

An aspect of the present technology relates to an IMD comprising a sensing circuitry configured to generate a biological signal representative of a physiologic parameter of a patient. The IMD also comprises a memory storing specific executable instructions and a denoising prediction model trained to predict a denoised biological signal segment from a noisy biological signal segment. The IMD further comprises a processor connected to the sensing circuitry and the memory and configured to execute the specific executable instructions to normally analyze the biological signal generated by the sensing circuitry to detect a characteristic of interest (COI) indicative of a pathophysiologic condition of the patient. The processor is also configured to denoise, when the IMD is exposed to a noise causing condition, noisy biological signal segments of the biological signal generated by the sensing circuitry while the IMD is exposed to the noise causing condition using the denoising prediction model to obtain denoised biological signal segments. The processor is further configured to analyze the denoised biological signal segments to detect the COI indicative of the pathophysiologic condition of the patient.

Another aspect of the present technology relates to a system for denoising biological signals. The system comprises an IMD comprising a sensing circuitry configured to generate a biological signal representative of a physiologic parameter of a patient. The IMD also comprises a first communication circuitry configured to perform wireless communication with an external device. The IMD further comprises a first memory storing specific executable instructions and a first processor connected to the sensing circuitry, the first communication circuitry and the first memory and configured to execute the specific executable instructions to normally analyze the biological signal sensed by the sensing circuitry to detect a COI indicative of a pathophysiologic condition of the patient. The first processor is also configured to control, when the IMD is exposed to a noise causing condition, the first communication circuitry to transmit, to the external device, a biological signal generated by the sensing circuitry while the IMD is exposed to the noise causing condition, or biological signal segments thereof. The external device comprises a second communication circuitry configured to perform wireless communication with the IMD. The external device also comprises a second memory storing specific executable instructions and a denoising prediction model trained to predict a denoised biological signal segment from a noisy biological signal segment. The external device further comprises a second processor connected to the second communication circuitry and the second memory and configured to execute the specific executable instructions to denoise, using the denoising prediction model, noisy biological signal segments of the biological signal, or of the biological signal segments thereof, received by the second communication circuitry from the IMD to obtain denoised biological signal segments. The second processor is also configured to control the second communication circuitry to transmit, to the IMD, the denoised biological signal segments or information of the COI indicative of the pathophysiologic condition of the patient detected by analysis of the denoised biological signal segments.

A further aspect of the present technology relates to a method for denoising biological signals. The method comprises an IMD generating a biological signal representative of a physiologic parameter of a patient. The IMD normally analyzing the biological signal to detect a COI indicative of a pathophysiologic condition of the patient. The method also comprises denoising, when the IMD is exposed to a noise causing condition, noisy biological signal segments of the biological signal generated while the IMD is exposed to the noise causing condition using a denoising prediction model to obtain denoised biological signal segments. The denoising prediction model is trained to predict a denoised biological signal segment from a noisy biological signal segment. The method further comprises analyzing the denoised biological signal segments to detect the COI indicative of the pathophysiologic condition of the patient.

The present technology thereby enables an IMD to perform analysis of biological signals generated while the IMD is exposed to a noise causing condition, such as exposed to electromagnetic interference (EMI) during an MRI session. This means that the IMD can perform monitoring and analysis of the generated biological signal for the purpose of detecting any COI indicative of a pathophysiologic condition also during such an MRI session. This significantly improves the workflow during MRI scanning of patients who have IMDs since it relaxes the need of monitoring the patients during MRI sessions by the referring physician/clinic staff, staff of the MRI clinic, staff of the implant clinic, and the implant manufacturer's staff. This also significantly reduces adjustments needed to be mode to operations modes of IMDs before and after MRI sessions.

### BRIEF DESCRIPTION OF THE DRAWINGS

The embodiments, together with further objects and advantages thereof, may best be understood by making reference to the following description taken together with the accompanying drawings, in which:
Fig. 1 illustrates a system comprising an IMD and an external device according to an embodiment;
Fig. 2 illustrates a leadless pacemaker according to an embodiment;
Fig. 3 illustrates a block diagram of an IMD according to an embodiment;
Fig. 4 illustrates a block diagram of an external device according to an embodiment;
Fig. 5 illustrates a patient with an IMD undergoing an MRI scanning session;
Fig. 6 is a flow chart illustrating a method for denoising biological signals according to an embodiment;
Fig. 7 is an illustration of using a denoising prediction model to denoise cardiac activity signal segments during an MRI session according to an embodiment;
Fig. 8 is an illustration of using a segment classification model to classify cardiac activity signal segments and a denoising prediction model to denoise cardiac activity signal segments during an MRI session according to an embodiment;
Fig. 9 is an illustration of an unfiltered MRI-induced gradient noise signal segment (top) and a filtered MRI-induced gradient noise signal segment used in generating training data sets for a denoising prediction model and for a segment classification model;
Fig. 10 is an illustration of a clean cardiac activity signal segment (top), a filtered MRI-induced gradient noise signal segment (middle) and a noisy cardiac activity signal segment (bottom) used in generating training data sets for a denoising prediction model and for a segment classification model;
Figs. 11A and 11B illustrate performance of a trained denoising prediction model according to an embodiment in the form of (A) ratio indicating similarity between original and denoised cardiac activity signal segments and (B) lag at maximum cross-correlation;
Figs. 12A and 12B illustrate examples of noisy cardiac activity signal segments input into a denoising prediction model according to an embodiment, denoised cardiac signal segments output by the denoising prediction model and corresponding clean cardiac activity signal segments;
Fig. 13 schematically illustrates a clean IEGM signal segment and a version of the clean IEGM signal segment denoised by a denoising prediction model according to an embodiment;
Fig. 14 illustrates clean cardiac data and noisy cardiac data used as training data for a denoising prediction method according to an embodiment;
Figs. 15A and 15B illustrate performance of a trained denoising prediction model according to an embodiment in the form of (A) ratio indicating similarity between original and denoised cardiac activity signal segments and (B) lag at maximum cross-correlation;
Figs. 16A and 16B illustrate examples of noisy cardiac activity signal segments input into a denoising prediction model according to an embodiment, denoised cardiac signal segments output by the denoising prediction model and corresponding clean cardiac activity signal segments; and
Fig. 17 is a flow chart illustrating a method for training a denoising prediction model according to an embodiment.

### DETERAILED DESCRIPTION

The present technology generally relates to methods, implantable medical devices (IMDs) and systems comprising IMDs, and in particular to such methods, IMDs and systems for denoising biological signals sensed by the IMDs.

The term "biological signal" includes signals, such as electrical signals, measured by one or more electrodes or other types of physiological sensors of or coupled to an IMD within a patient. The biological signal is representative of a physiological parameter of the patient, such as indicative of a cardiac activity characteristic, hemodynamic characteristic and/or body generated analyte. The biological signals are susceptible to interference induced by a noise causing condition, such as magnetic resonance imaging (MRI) induced interference. Illustrative, but non-limiting, examples of biological signals include cardiac activity (CA) signals, neurological signals, cardiac impedance signals, pulmonary impedance signals, transthoracic impedance signals, accelerometer signals, temperature signals, venous oxygen saturation (SvO2) signals, electrical photoplethysmography (PPG) signals, heart sounds, pulmonary arterial pressure signals, blood pressure signals, and the like. Examples of CA signals include electrocardiogram (ECM) signals, electrogram (ECG) signals, and cardiac impedance signals, but are not limited thereto. The biological signals, with which the denoising disclosed herein should be used, do not include signals that are not susceptible to interference induced by noise causing conditions. As an illustrative example, optical PPG signals, in which a light source emits light to a tissue and a photodetector measures the reflected light from the tissue, are generally not susceptible to MRI induced interference.

The term "pathophysiologic condition" is a non-physiologic condition indicating an abnormal or unhealthy state of a patient condition of interest. This should be compared to a non-pathophysiologic condition or physiologic condition that indicates a normal or healthy state of the patient condition of interest. Non-limiting examples of patient conditions of interest include electrical or hemodynamic cardiac behavior, for instance normal sinus rhythms as a physiologic condition and arrhythmias or unstable hemodynamic performance as examples of pathophysiologic conditions, neurological behavior, for instance pain, tremors, Parkinson's disease, tinnitus, Alzheimer's disease, and other neurological disorders measurable and treatable within the spine, brain and peripheral muscles as illustrative examples of pathophysiologic conditions, blood pressure, pulse oximetry levels, diabetes and other conditions due to imbalances or deficiencies of body generated analytes.

The term "characteristic of interest (COI)" as used herein is a COI indicative of a pathophysiologic condition of a patient and where the COI can be detected by analysis of the biological signal. Such a COI could, for instance, be in the form of an abnormal heart rhythm, i.e., an arrhythmia, as detected by analysis of a CA signal, or in the form of an abnormal body temperature, SvO2 level, heart sound, pulmonary arterial pressure, etc. depending on the particular biological signal.

Generally, an IMD comprises a sensing circuitry configured to generate a biological signal representative of a physiologic parameter of a patient. The IMD can thereby analyze the biological signal to monitor the health of the patient and detect any COI indicative of a pathophysiologic condition of the patient. The IMD may then take actions upon such a detection of the COI indicative of the pathophysiologic condition, such as apply a treatment of the pathophysiologic condition and/or provide diagnostic information indicative of the pathophysiologic condition to the patient or a physician to thereby inform the patient or the physician of the detected pathophysiologic condition.

Such an analysis of the biological signal for the purpose of monitoring the health of the patient and detect any COI indicative of a pathophysiologic condition generally requires the biological signal to be substantially free from interference and noise in order for the analysis of the biological signal to be reliable and accurate. Accordingly, if the IMD is exposed to a noise causing condition that induces interference or noise onto the biological signal generated by the sensing circuitry of the IMD, then the noisy biological signal does not reliably represent a physiologic parameter of the patient due to the presence of interference or noise in the biological signal. This means that if the noisy biological signal would have been analyzed and used to detect any COI then such an analysis would be unreliable and cannot be used to accurately monitor for any COI indicative of a pathophysiologic condition. In such a situation, the IMD is typically configured to temporarily disable the generation and/or analysis of the biological signal. As an illustrative example, strong MRI fields from an MR scanner can prevent an IMD from reliably sensing signals from the various electrodes and from the various physiological sensors, resulting in improper and/or dangerous operation if the IMD is basing its operation on biological signals generated and analyzed while exposed to such MRI fields. Accordingly, MRI conditional IMDs are placed in an MRI mode and/or programmed with MRI settings temporarily limiting or disabling the IMD's normal operation. This, however, means that the IMD will no longer monitor the biological signal during an MRI session. In clear contrast, personnel need to be present and monitor the health of the patient during the MRI session as the monitoring has been temporarily disabled in the IMD. This further typically requires personnel with expertise in the IMD to be on-site at the MRI clinic in order to place the IMD in the MRI mode and then, once the MRI session is over, restore the normal operation of the IMD.

The present technology solves the above-mentioned shortcomings of the prior art by denoising the noisy biological signal using a denoising prediction model. This means that the IMD can continue to generate and analyze the biological signal even when exposed to interference or noise by a noise causing condition, such as during an MRI session, by denoising the noisy biological signal prior to analysis. The so-obtained denoised biological signal can then be analyzed to detect any COI indicative of a pathophysiologic condition of the patient. The technology thereby reduces the need for reprogramming the IMD into an MRI mode during an MRI session, which thereby simplifies the workflow for MRI scanning of patients who have IMDs while ensuring the safety of the patients since the IMDs are able to reliably and accurately monitor biological signals even if interference or noise is present therein by usage of the denoising prediction model.

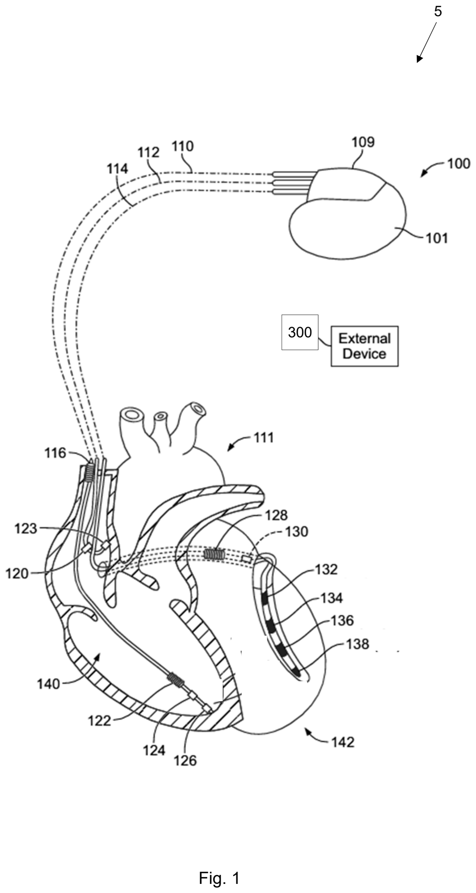

Fig. 1 illustrates a system 5 comprising an IMD 100 and an external device 300 according to an embodiment. In this illustrative example, the IMD 100 is coupled to a heart 142 in a patient and configured to sense cardiac activity (CA) signals using at least some of the electrodes 120, 123, 124, 126, 130, 132, 134, 136, 138 present on one or more cardiac leads 110, 112, 114. The IMD 100 is one example of the type of device that may collect CA signals as biological signals. The external device 300 may be a programmer, an external defibrillator, a workstation, a portable computer, a personal digital assistant, a cell phone, a bedside monitor and the like. The IMD 100 of Fig. 1 may be in the form of a cardiac monitoring device, a pacemaker, a cardioverter, a cardiac rhythm management device, a defibrillator, or the like, implemented in accordance with an embodiment of the present technology. The IMD 100 may be a dual-chamber stimulation device capable of treating both fast and slow arrhythmias with stimulation therapy, including cardioversion, defibrillation, anti-tachycardia pacing and pacing stimulation, as well as capable of detecting heart failure, evaluating its severity, tracking the progression thereof, and controlling the delivery of therapy and warnings in response thereto. The IMD 100 may be controlled to sense atrial and ventricular waveforms of interest, discriminate between two or more ventricular waveforms of interest, deliver stimulus pulses or shocks, and inhibit application of a stimulation pulse to the heart 142 based on the discrimination between the waveforms of interest and the like.

The IMD 100 generally includes a housing 101 that is joined to a header assembly 109 that holds receptacle connectors connected to a right ventricular lead 110, a right atrial lead 112, and a coronary sinus lead 114, respectively. The leads 110, 112, 114 measure cardiac signals of the heart 142. The right atrial (RA) lead 112 includes an RA tip electrode 123 and an RA ring electrode 120. The coronary sinus lead 114 includes a left atrial (LA) ring electrode 130, a LA coil electrode 129 and one or more left ventricular (LV) electrodes 132, 134, 136, 138 to form a multi-pole LV electrode combination. The right ventricular (RV) lead 110 is configured for implantation in the right ventricle 140 of the heart 142 and includes an RV tip electrode 126, an RV ring electrode 124, an RV coil electrode 122, and a superior vena cava (SVC) coil electrode 116. The leads 112, 114, 116 can be used to detect intracardiac electrogram (IEGM) signals that are processed and analyzed as described herein. The leads 112, 114, 116 may also delivery therapies to the heart 142.

The IMD 100 of Fig. 1 is an illustrative, but non-limiting, example of an IMD 100 according to the embodiments. The IMD 100 does not necessarily have to monitor biological signals from the heart 142 of the patient but could alternatively be designed to monitor and optionally provide therapy to other tissues or organs in a patient. Thus, other examples of IMDs 100 include, but not limited to, neurostimulators, gastric stimulators, and the like.

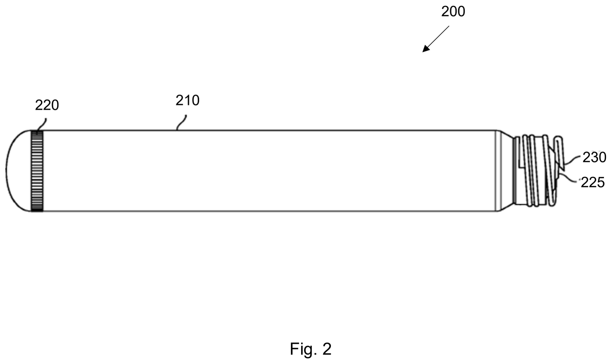

Further, the IMD 100 does not necessarily have to be coupled to one or more leads, such as cardiac leads 110, 112, 114 as illustrated in Fig. 1. The IMD 200 could, for instance, be in the form of a leadless device, such as a leadless (cardiac) pacemaker as illustrated in Fig. 2. The leadless pacemaker 200 is substantially enclosed in a hermetic housing 210 suitable for placement on or attachment to the inside or outside of a cardiac chamber, such as the right atrium and/or right ventricle of the patient's heart, but not limited thereto. Attachment of a leadless pacemaker 200 to the cardiac tissue can be accomplished via a helical anchor or helix 230 on an anchor mount extending from a distal end of the leadless pacemaker 200.

The leadless pacemaker 200 can have two or more electrodes 220, 225 located within, on, or near the housing 210, for sensing electrical activity from the heart, and preferably for bidirectional communication with at least one other device within or outside the body, and optionally for delivering pacing pulses to the cardiac muscle of the cardiac chamber. The housing 210 can contain a battery to provide power for pacing, sensing, and communication. The housing 210 may also contain sensing circuitry for sensing cardiac activity from the electrodes 220, 225. The housing 210 may contain communication circuit for receiving information from at least one other device via the electrodes 220, 225 and optionally contains pacing circuitry for generating pacing pulses for delivery via the electrodes 220, 225.

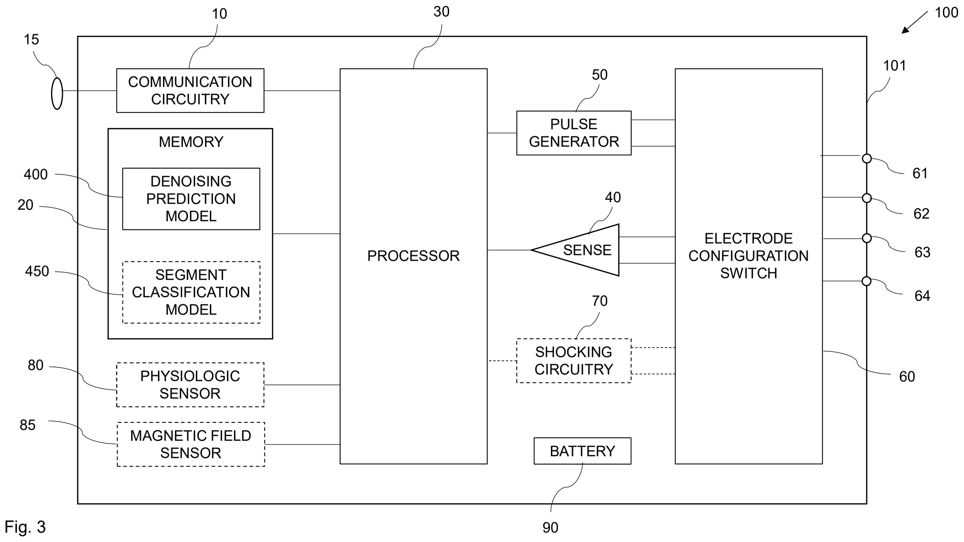

An aspect of the present technology will now be described in more detail with reference to the block diagram of Fig. 3. Fig. 3 illustrates an example of an IMD 100, such as the IMD 100 as shown in Fig. 1. The disclosure of the present technology is, however, not limited to the particular IMD 100 as shown in Fig. 1 capable of sensing and preferably pacing a patient's heart but may likewise apply to other types of IMDs, including leadless IMDs 200 as shown in Fig. 2, and further mentioned in the foregoing.

An aspect of the present technology relates to an IMD 100 comprising a sensing circuitry 40 configured to generate a biological signal representative of a physiologic parameter of a patient. The IMD 100 also comprises a memory 20 storing specific executable instructions and a denoising prediction model 400. The denoising prediction model 400 is trained to predict a denoised biological signal segment from a noisy biological signal segment. The IMD 100 further comprises a processor 30 connected to the sensing circuitry 40 and the memory 20 and configured to execute the specific executable instructions to normally analyze the biological signal generated by the sensing circuitry 40 to detect a COI indicative of a pathophysiologic condition of the patient. The processor 30 is also configured to denoise, when the IMD 100 is exposed to a noise causing condition, noisy biological signal segments of the biological signal generated by the sensing circuitry 40 while the IMD 100 is exposed to the noise causing condition using the denoising prediction model 400 to obtain denoised biological signal segments. The processor 30 is further configured to analyze the biological signal segments to detect the COI indicative of the pathophysiologic condition of the patient.

The IMD 100 of the present technology thereby comprises sensing circuitry 40 configured to generate the biological signal. This biological signal is normally, i.e., when the IMD 100 is not exposed to the noise causing condition, analyzed by the processor 30 to detect any COI indicative of the pathophysiologic condition of the patient. Conventionally, when the IMD 100 is exposed to a noise causing condition, the IMD 100 is typically put into a noise operating mode, such as represented by the above-mentioned MRI mode. In such a case, the sensing circuitry 40 is temporarily disabled from sensing the biological signal and/or the processor 30 is temporarily disabled from analyzing the noisy biological signal as long as the IMD 100 is exposed to the noise causing condition. However, in the present technology, the IMD 100 does not need to be put in the noise operating mode when exposed to the noise causing condition or, if still put in the noise operating mode when exposed to the noise causing condition, the sensing circuitry 40 can continue to generate the biological signal even when the IMD 100 is exposed to the noise causing condition. In such a case, rather than analyzing the noisy biological signal as generated by the sensing circuitry 40, the processor 30 first denoises the noisy biological signal by inputting one or more noisy biological signal segments of the noisy biological signal into the denoising prediction model 400, which predicts corresponding denoised biological signal segments from the input noisy biological signal segments. The processor 30 can thereby analyze these denoised biological signal segments to detect the COI indicative of the pathophysiologic condition of the patient.

In other words, the processor 30 of the IMD 100 normally, i.e., when the IMD 100 is not exposed to any noise causing condition, analyzes the biological signal as generated by the sensing circuitry 40. However, when the IMD 100 is exposed to a noise causing condition, the processor 30 first predicts, using the denoising prediction model 400, denoised biological signal segments from noisy biological signal segments of the noisy biological signal generated by the sensing circuitry 40 while the IMD 100 is exposed to the noise causing condition. This means that during normal operation, i.e., without exposure to noise or interference, the analysis by the processor 30 is made on the biological signal generated by the sensing circuitry 40, whereas during noisy operation, i.e., with exposure to noise or interference, the analysis by the processor 30 is made on denoised biological signal segments predicted by the denoising prediction model 400.

The decision whether the IMD 100 is exposed to a noise causing condition and thereby should denoise the biological signal prior to analysis thereof can be made according to various embodiments.

In an embodiment, the IMD 100 comprises a communication circuitry 10 configured to receive a noise mode programming command from an external device. In such an embodiment, the processor 30 is connected to the communication circuitry 10 and is configured to execute the specific executable instructions to denoise, in response to reception of the noise mode programming command, the noisy biological signal segments using the denoising prediction model 400 to obtain the denoised biological signal segments.

In this embodiment, an external device capable of wirelessly communicating with the IMD 100, and in particular by the communication circuitry 10 thereof, is used to transmit a noise mode programming command to the IMD 100. Such a noise mode programming command may, for instance, be in the form of an MRI mode programming command currently used to put MRI conditional IMDs in an MRI mode during an MRI session. However, rather than disabling the sensing circuitry 40 or disabling analysis of the biological signal sensed by the sensing circuitry 40 as in the prior art in response to such an MRI mode programming command, the processor 30 is triggered by the MRI mode programming command to denoise the noisy biological signal segments using the denoising prediction model 400 to obtain the denoised biological signal segments.

This embodiment thereby reuses the MRI mode signaling traditionally used to set MRI conditional IMDs in a safe mode during an MRI session in order to notify the IMD 100 and the processor thereof 30 when the IMD 100 is exposed to the noise causing condition, in this embodiment represented by electromagnetic interference (EMI) induced by the MR scanner during an MRI session.

In an embodiment, the communication circuitry 10 is also configured to receive a normal mode programming command from the external device, such as at the end of the MRI session. In such a case, the processor 30 is configured to execute the specific executable instructions to analyze, in response to reception of the normal mode programming command, the biological signal generated by the sensing circuitry 40 to detect the COI indicative of the pathophysiologic condition of the patient.

Thus, the normal mode programming command is used by the processor 30 to switch back to normal operation mode and thereby analyze the biological signal generated by the sensing circuitry 40 without the need for any denoising using the denoising prediction model 400.

In an alternative, or additional embodiment, the processor 30 is configured to execute the specific executable instructions to analyze the biological signal generated by the sensing circuitry 40 during a noise-sampling period to detect noise or interference in the biological signal. The processor 30 is, in this embodiment, also configured to denoise, in response to detection of noise or interference in the biological signal, the noisy biological signal segments using the denoising prediction model 400 to obtain the denoised biological signal segments.

This embodiment thereby utilizes the noise-detection capability of the processor 30 to determine whether the IMD 100 is exposed to a noise causing condition. In this embodiment, the processor 30 is preferably configured to execute a noise-detection algorithm that could be used to detect the presence of noise or interference on the biological signal. As an example, IMDs 100 couplable to one or more implantable leads 110, 112, 114 as shown in Fig. 1 typically employ an algorithm to verify lead integrity and thereby detect a situation with lead failure. Hence, the processor 30 could be configured to analyze the biological signal during a noise-sampling period to detect any lead failure that may cause noise or interference in the biological signal. Various such lead failure or dysfunction detection algorithms are known in the art including, but not limited to, LIA (Lead Integrity AlertTM) that is based on monitoring for changes in lead impedance, dynamic noise algorithms that detect noise or interference on the lead signals by analyzing the frequency and amplitude of the generated biological signal, short interval detection that identifies lead failure by detecting very short intervals between sensed cardiac activity that can indicate a fracture or insulation breach in the implantable lead, morphology analysis that analyzes the shape and characteristics of the sensed biological signal to detect abnormalities that may indicate lead failure, etc. Another example of noise detection algorithm is to recognize patterns of repetitive signals at high frequencies, such as above a threshold frequence of, for instance, 20 Hz, 25 Hz, 50 Hz, 75 Hz or 100 Hz. When the processor 30 detects such repetitive signals sustained long enough, the processor 30 concludes that the biological signal comprises noise or interference. The noise or interference causing source could, for instance be EMI, myopotential oversensing, etc.

In an embodiment, the processor 30 is configured to switch from denoising noisy biological signal segments using the denoising prediction model 400 to analyzing the biological signal as generated by the sensing circuitry 40 when the processor 30 no longer detects any noise or interference in the biological signal during a noise-sampling period.

In an alternative, or additional embodiment, the IMD 100 comprises a communication circuitry 10 connected to a communication coil 15. In such an embodiment, the processor 30 is connected to the communication circuitry 10 and is configured to execute the specific executable instructions to monitor, when the IMD 100 is exposed to the noise causing condition, for noise on the communication coil 15. The processor 30 is also configured to denoise, when noise is detected on the communication coil 15, the noisy biological signal segments using the denoising prediction model 400 to obtain the denoised biological signal segments.

This embodiment is useful if the IMD 100 comprises a communication coil 15, sometimes referred to as telemetry coil in the art, that is used by the IMD 100 to perform wireless communication with external devices. In such a case, the noise causing condition, such as EMI, may induce noise on the communication coil 15. The processor 30 is then configured to monitor for any such noise on the communication coil 15 and, in response to detection of such noise, denoise the noise biological signal segments using the denoising prediction model 400. In this embodiment, the processor 30 is preferably configured to switch back from denoising noisy biological signal segments to directly analyzing the biological signal generated by the sensing circuitry 40 when the processor 30 no longer detects any noise on the communication coil 15.

A further alternative, or additional, embodiment of detecting the noise causing condition is to use a magnetic field sensor 85. In such an embodiment, the IMD 100 comprises a magnetic field sensor 85 configured to detect a magnetic field. The processor 30 is connected to the magnetic field sensor 85 and is configured to execute the specific executable instructions to denoise, in response to the magnetic field sensor 85 detecting the magnetic field, the noisy biological signal segments using the denoising prediction model 400 to obtain the denoised biological signal segments.

This embodiment can be used by the IMD 100 to detect the presence of a magnetic field, such as in connection with an MRI session. If such a magnetic field is detected by the magnetic field sensor 85, the processor 30 is triggered to switch from analyzing the biological signal generated by the sensing circuitry 40 to first denoise the noisy biological signal segments of the noisy biological signal sensed by the sensing circuitry 40 while the magnetic field sensor 85 senses the magnetic field.

The IMD 100 could be operated according to any of the above-described embodiments. An IMD 100 may also have access to multiple of the above-described embodiments and can thereby detect a noise-causing condition in more than one way.

In an embodiment, the denoising prediction model 400 used by the processor 30 to predict denoised versions of noisy biological signal segments receives a noisy biological signal segment as input and predicts a denoised biological signal segment therefrom.

In another embodiment, the denoising prediction model 400 may use other inputs in addition to the noisy biological signal segments to predict denoised biological signal segments. An example of such an additional input is any voltage induced on a communication coil 15 by EMI, such as MRI-induced gradient voltage. In such an embodiment, the IMD 100 comprises a communication circuitry 10 connected to a communication coil 15. The processor 30 is then connected to the communication circuitry 10 and is configured to execute the specific executable instructions to determine, when the IMD 100 is exposed to EMI, an EMI-induced voltage on the communication coil 15. The processor 30 is, in this embodiment, also configured to denoise, when the IMD 100 is exposed to the EMI, the noisy biological signal segments to obtain the denoised biological signal segments by inputting the noisy biological signal segments and the determined EMI-induced voltage on the communication coil 15 into the denoising prediction model 400.

In an example embodiment, the processor 30 is configured to monitor any EMI-induced voltage on the communication coil 15. The processor 30 then preferably generates a voltage signal representative of the EMI-induced voltage over time. In such a case, the processor 30 inputs a voltage signal segment together with a noisy biological signal segment into the denoising prediction model 400. The voltage signal segment and the noisy biological signal segment preferably overlap at least partly timewise. In other words, the voltage signal segment preferably represents the voltage determined or measured on the communication coil 15 during the same period of time as the sensing circuitry 40 generates the corresponding noisy biological signal segment. Hence, it is generally preferred if the voltage signal segment and the noisy biological signal segment input together into the denoising prediction model 400 are time aligned. Such a time alignment can be achieved by using time stamps for the biological signal generated by the sensing circuitry 40 and for the voltage signal generated by the processor 30. Hence, in a preferred embodiment, two time-aligned signal segments are input to the denoising prediction model 400 and used by the model 400 to predict a corresponding denoised biological signal segment.

In another example embodiment, the processor 30 does not necessarily generate a voltage signal representative of the EMI-induced voltage on the communication coil 15 over time. Alternatively, the processor 30 could determine a voltage parameter representative of the EMI-induced voltage on the communication coil 15, such as during a time period corresponding to a duration of a noisy biological signal segment. As an example of such a voltage parameter is an average EMI-induced voltage during the time period corresponding to a duration of a noisy biological signal segment. Alternatively, the average EMI-induced voltage could be complemented with a parameter representing the distribution of the EMI-induced voltage during the time period, such as the standard deviation. In such an embodiment, the processor 30 inputs the at least one voltage parameter, such as average EMI-induced voltage and optionally standard deviation, together with a noisy biological signal segment into the denoising prediction model 400, which then predicts a corresponding denoised biological signal segment based on the at least one voltage parameter and the noisy biological signal segment.

When the IMD 100 is exposed to the noise causing condition, noise or interference may be present on the biological signal from time to time, such as intermittently. Thus, the noise causing condition may induce noise or interference on some biological signal segments, i.e., noisy biological signal segments, whereas other segments of the biological signal do not contain any such noise or interference, i.e., non-noisy or clean biological signal segments. As an illustrative example, during an MRI session, the MR scanner is not scanning during the whole MRI session, rather the MR scanner is "off" between scanning sequences. Thus, during such time periods between scanning sequences, the IMD is not exposed to any EMI induced by the MR scanner. Thus, a biological signal generated during at least a part of an MRI session may contain noisy biological signal segments as generated when the MR scanner is "on", i.e., during an ongoing scanning sequence, and non-noisy or clean biological signal segments as generated when the MR scanner is "off", i.e., between scanning sequences.

The denoising prediction model 400 is trained based on noisy biological signal segments to predict denoised versions thereof. Accordingly, the denoising prediction model 400 is thereby generally not trained to handle clean biological signal segments without any noise or interference. Thus, the analysis of non-noisy or clean biological signal segments for the purpose of detecting any COI indicative of the pathophysiologic condition of the patient is typically more accurate if performed directly on the non-noisy biological signal segment as compared to a denoised version of the non-noisy biological signal segment. Thus, in an embodiment, it would be beneficial to identify those biological signal segments that are noisy or non-noisy, i.e., clean, in the biological signal generated by the sensing circuitry 40 while the IMD 100 is exposed to the noise causing condition. In such a case, only those biological signal segments classified as noisy should be input into the denoising prediction model 400, whereas the non-noisy or clean biological signal segments could be used directly by the processor 30 without any denoising.

In an embodiment, the memory 20 comprises a segment classification model 450 trained to classify biological signal segments as noisy or non-noisy biological signal segments. The processor 30 is, in this embodiment, configured to execute the specific executable instructions to segment, while the IMD 100 is exposed to the noise causing condition, the biological signal generated by the sensing circuitry 40 while the IMD 100 is exposed to the noise causing condition into a plurality of biological signal segments. The processor 30 is, in this embodiment, also configured to classify the plurality of signal segments using the segment classification model 450. The processor 30 is further configured to denoise the biological signal segments classified as noisy by the segment classification model using the denoising prediction model 400 to obtain denoised biological signal segments.

Hence, in this embodiment, the IMD 100 has access to two models 400, 450. One of the models 400, 450 is trained to classify biological signal segments as noisy or non-noisy, i.e., clean, and the other of the models 400, 450 is trained to predict a denoised version of a noisy biological signal segment. It is preferred in this embodiment to merely denoise those biological signal segments of the biological signal that are classified by the segment classification model 450 as being noisy, i.e., predicted to contain noise or interference induced by a noise causing condition. Hence, in an embodiment, the processor 40 is configured to analyze the denoised biological signal segments and biological signal segments classified as non-noisy or clean by the segment classification model to detect the COI indicative of the pathophysiologic condition of the patient.

As described in the foregoing, the denoising prediction model 400 could use a determined EMI-induced voltage, such as in the form of a voltage signal segment, as input in addition to a noisy biological signal segment. Such a determined EMI-induced voltage may also, or alternatively, be used as input into the segment classification model 450.

In an embodiment, the IMD 100 comprises a communication circuitry 10 connected to a communication coil 15. The processor 30 is connected to the communication circuitry 10 and is configured to execute the specific executable instructions to determine, when the IMD 100 is exposed to EMI, an EMI-induced voltage on the communication coil 15. The processor 30 is, in this embodiment, also configured to classify the plurality of biological signal segments as noisy or non-noisy biological signal segments by inputting the plurality of biological signal segments and the determined EMI-induced voltage on the communication coil 15 into the segment classification model 450.

The various embodiments described in the foregoing about using an EMI-induced voltage as input to the denoising prediction model 400 also apply to using the EMI-induced voltage as input to the segment classification model 450.

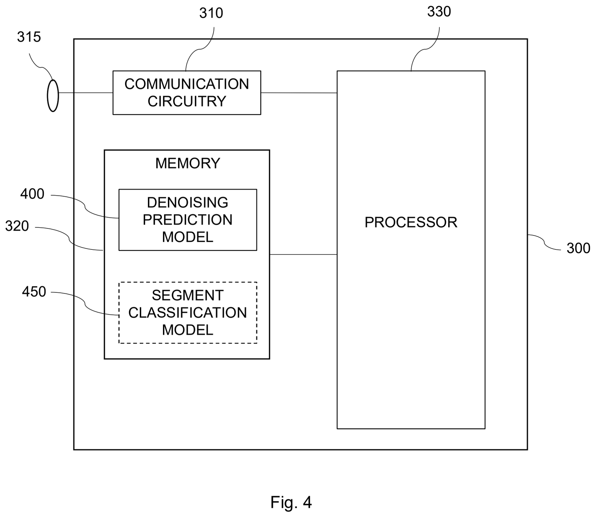

In the above-described embodiments, the segment classification is performed by a segment classification model 450 implemented locally at the IMD 100, such as in the memory 20 of the IMD 100. Such a local implementation of the model 450 enables a real-time or at least near real-time signal segment classification and thereby analysis of the processor 30 to detect any COI indicative of a pathophysiologic condition of the patient in (near) real-time, i.e., without any significant delays. This, however, comes at the expense of performing the optional segment classification in the IMD 100, which consumes provided power by a battery 90 of the IMD 100, which can reduce the longevity of the IMD 100. In some applications it might be more beneficial to implement the computations by the segment classification model 450 remotely, i.e., in an external device, such as in an external device 300 in Fig. 4.

In such an embodiment, see Figs. 3 and 4, the IMD 100 comprises a communication circuitry 10 configured to transmit, to an external device 300 comprising a segment classification model 450 trained to classify biological signal segments as noisy or non-noisy biological signal segments, the biological signal generated by the sensing circuitry 20 while the IMD 100 is exposed to the noise causing condition. Alternatively, the communication circuitry 10 is configured to transmit a plurality of biological signal segments of the biological signal to the external device 300. The communication circuitry 10 is, in this embodiment, also configured to receive, from the external device 300, a respective classification of the plurality of biological signal segments as noisy or non-noisy. In such an embodiment, the processor 30 is connected to the communication circuitry 10 and is configured to execute the specific executable instructions to denoise biological signal segments classified as noisy by the segment classification model 450 using the denoising prediction model 400 to obtain denoised biological signal segments.

Hence, in this embodiment, the segment classification is done remotely in the external device 300, which then comprises a memory 320 comprising the segment classification model 450, whereas the denoising prediction is done locally in the IMD 100 using the denoising prediction model 400. This embodiment thereby reduces the computations performed by the IMD 100 to classify biological signal segments when exposed to a noise causing condition and thereby reduces the power consumption by the battery 90 but at the cost of a slight delay in the form of transmitting the biological signal, or biological signal segments thereof, to the external device 300 and then receiving the classification information therefrom.

As discussed in the foregoing for the case of a locally implemented segment classification model 450, the segment classification model 450 can use an additional input in the form of an EMI-induced voltage. Such an embodiment can also be used for the remotely implemented segment classification model 450. In such an embodiment, the IMD 100 comprises a communication coil 15 connected to the communication circuitry 10. The processor 30 is, in this embodiment, configured to execute the specific executable instructions to determine, when the IMD 100 is exposed to EMI, an EMI-induced voltage on the communication coil 15. The communication circuitry 10 is, in this embodiment, configured to transmit, to the external device 300, the biological signal sensed by the sensing circuitry while the IMD 100 is exposed to the EMI, or the plurality of biological signal segments thereof, and the determined EMI-induced voltage on the communication coil 15. The external device 300 then inputs both the EMI-induced voltage, such as in the form of a voltage signal segment, and a biological signal segment into the segment classification model 450 to thereby classify the input biological signal segment as noisy or non-noisy.

The various embodiments described in the foregoing about using an EMI-induced voltage as input to the denoising prediction model 400 applies also to using the EMI-induced voltage as input to the segment classification model 450.

In some embodiments the IMD 100 may detect a noisy condition on the communication coil 15 and/or the magnetic field sensor 85. For instance, detection of an EMI-induced voltage on the communication coil 15 could be detected by the processor 30 of the IMD 10, as indication of presence of a noise causing condition. Alternatively, or in addition, the processor 30 could use the magnetic field sensor 85 to detect the noise causing condition. In such a case, the processor 30 could monitor the communication coil 15 and/or the magnetic field sensor 85 while the sensing circuitry 40 generates the biological signal representative of a physiologic parameter of a patient. In such an embodiment, the processor 30 could perform the classification of biological signal segments as noisy or clean biological signal segments based on the presence of any EMI-induced voltage on the communication coil 15 and/or detection of presence of a noise causing condition by the magnetic field sensor 85. In particular, a biological signal segment sensed by the sensing circuitry 40 while the processor 30 detects the presence of a noise causing condition, such as by monitoring the communication coil 15 and/or the magnetic field sensor 85, could be classified by the processor 30 as a noisy biological signal segment. Correspondingly, a biological signal segment sensed by the sensing circuitry 40 while the processor 30 does not detect any noise causing condition, such as by monitoring the communication coil 15 and/or the magnetic field sensor 85, could be classified by the processor 30 as a clean, i.e., non-noisy, biological signal segment. Time stamps of the biological signals could then be used together with any time stamps of the sensor signal from the magnetic field sensor 85 and/or time stamps of detected EMI EMI-induced voltage signal on the communication coil 15 to identify those biological signal segments that coincide with the presence of a noise causing condition.

This embodiment could be used as an alternative or complement to the segment classification model 450.

The IMD 100 may be implemented as a full-function biventricular pacemaker, equipped with both atrial and ventricular sensing and pacing circuitry for four chamber sensing and stimulation therapy (including both pacing and shock treatment). Optionally, the IMD 100 may provide full-function cardiac resynchronization therapy. Alternatively, the IMD 100 may be implemented with a reduced set of functions and components. For instance, the IMD 100 may be implemented with atrial sensing and pacing but without ventricular sensing and pacing. For another example, the IMD 100 may be implemented with ventricular sensing and pacing but without atrial sensing and pacing. For still another example, the IMD 100 can be an implantable cardiac monitor (ICM), also known as an insertable cardiac monitor, that includes the sensing circuitry 40 configured to generate a biological signal representative of a physiologic parameter of a patient, but does not provide any therapy. Such an ICM may perform denoising of biological signal segments and analyze the denoised biological signal segments to detect a COI indicative of a pathophysiologic condition of the patient, and may store in its memory and/or upload to an external device data indicative of the COI detected by the ICM.

Generally, the IMD 100 has a housing 101 to hold the electronic/computing components. The housing 101, which is often referred to as the "can", "case", "encasing", or "case electrode", may be programmably selected to act as the return electrode for certain stimulus modes. The housing 101 further includes a connector 109, see Fig. 1, with a plurality of terminals 61, 62, 63, 64. The terminals 61, 62, 63, 64 may be connected to electrodes that are located in various locations within and about the heart 142. The type and location of each electrode may vary. For example, the electrodes may include various combinations of ring, tip, coil and shocking electrodes and the like. The IMD 100 preferably comprises an electrode configuration switch 60, which includes multiple switches for connecting the desired terminals 61, 62, 63, 64 and thereby electrodes to the appropriate circuits 40, 50, 70, thereby facilitating electrode programmability.

In an embodiment, the sensing circuitry 40 is couplable to at least one electrode to thereby sense an electrical signal from a tissue, in particular cardiac tissue, and configured to generate the biological signal based on the sensed electrical signal.

Thus, the sensing circuitry 40 is selectively coupled to one or more electrodes that is configured to obtain biological signals, e.g., CA signals, neurological activity signals, and the like, indicative of a physiologic parameter of a patient over a period of time. For example, the sensing circuitry 40 performs sensing operations through the electrode configuration switch 60 to detect the presence of cardiac activity in a chamber of the heart. The sensing circuitry 40 may include dedicated sense amplifiers, multiplexed amplifiers, or shared amplifiers. It may further employ one or more low power, precision amplifiers with programmable gain and/or automatic gain control, bandpass filtering, and threshold detection circuit to selectively sense the cardiac signal of interest. The output of the sensing circuitry 40 is connected to the processor 30, which analyzes the biological signal from the sensing circuitry 40 and, in turn, triggers or inhibits a pulse generator 50 in response to the absence or presence of cardiac activity. The sensing circuitry 40 preferably receives a control signal from the processor 30 for purposes of controlling the gain, threshold, polarization charge removal circuitry (not shown), and the timing of any blocking circuitry (not shown) coupled to the inputs of the sensing circuitry 40. While not specifically shown, one or more filters may be located upstream and/or downstream of the sensing circuitry 40 to filter the signals sensed by the sensing circuitry before the signals are provided to the processor 30.

The IMD 100 includes a processor 30, such as in the form of a programmable microcontroller, configured to control various operations of the IMD 100, including cardiac monitoring and stimulation therapy. The processor 30 includes one or more microprocessors or central processing units (CPUs), or equivalent control circuitry. The IMD 100 also includes memory 20, such as in the form of random access memory (RAM) and/or read-only memory (ROM), configured to store program instructions, i.e., specific executable instructions, executable by the processor 30 to perform the operations described herein, as well as the overall sensing, analyzing and therapy delivery functionality. The IMD 100 could include a single memory 20 as shown in Fig. 3 or multiple, i.e., at least two, memories, such as in the form of a memory bank.

In an embodiment, the IMD 100 further includes a pulse generator 50 that generates stimulation pulses for delivery by one or more electrodes coupled thereto. The pulse generator 50 may deliver pacing pulses, anti-tachycardia pacing therapy and the like. The pulse generator 50 is controlled by the processor 30 via a control signal. The pulse generator 50 is coupled to the select electrode(s) via the electrode configuration switch 60. Such electrode(s) can be coupled to the terminals 61, 62, 63, 64 and may be located on leads coupled to the IMD 100. Such electrode(s) may be implemented as part of the housing 101 of the IMD 100 and/or may be in close proximity to the housing 101, e.g., if the IMD is a leadless pacemaker or an ICM.

The processor 30 may, in an embodiment, include timing control circuitry to control the timing of the stimulation pulses, e.g., pacing rate, atrio-ventricular (AV) delay, atrial interconduction (A-A) delay, or ventricular interconduction (V-V) delay, etc. The timing control circuitry may also be used for the timing of refractory periods, blanking intervals, noise detection periods or windows, evoked response windows, alert intervals, marker channel timing, and so on.

In an embodiment, the processor 30 has an arrhythmia detector (not shown) for detecting arrhythmia conditions, and to review and analyze one or more features of the morphology of cardiac signals. Although not shown, the processor 30 may further include other dedicated circuitry and/or firmware/software components that assist in monitoring various conditions of the patient's heart and managing pacing therapies.

The operating parameters of the IMD 100 may be non-invasively programmed into the memory 20 through a communication circuitry 10, such as in telemetric communication via a communication link with an external device. The communication circuitry 10 may use high frequency modulation, for example using radio frequency (RF), Bluetooth^{®}, or Bluetooth Low Energy (BLE) telemetry protocols. The communication circuit 10 allows biological signals, or signal segments thereof, to be sent to the external device through the established communication link. The communication circuitry 10 could be implemented as a transceiver or a transmitter and receiver pair. The communication circuitry 100 may alternatively, or additionally, provide for conductive communication (also known as conducted communication) whereby communication may occur through the same electrodes that are used for sensing and/or delivery of pacing therapy.

In an embodiment, the IMD 100 comprises a magnetic field sensor 85 that is configured to detect a leading transition portion of the active field interval from at least one of the RF or gradient fields. For example, the magnetic field sensor 85 may be constructed as described in U.S. patent no. 8,200,334, U.S. patent publication nos. 2024/0203550 A1 or 2022/0339452 A1.

In an embodiment, the IMD 100 may further include one or more physiologic sensors 80. Such a physiologic sensor 80 could be in the form of a "rate-responsive" sensors that is typically used to adjust pacing stimulation rates according to the exercise state of the patient. However, the physiological sensor 80 may further, or alternatively, be used to detect changes in cardiac output, changes in the physiological condition of the heart, or diurnal changes in activity, e.g., detecting sleep and wake states. Biological signals generated by the physiological sensor 80 is passed to the processor 30 for analysis. The processor 30 may respond by adjusting the various pacing parameters, such as pacing rate, AV delay, V-V delay, etc., at which the atrial and/or ventricular pacing pulses are administered. While shown as being included within the IMD 100, the physiologic sensor(s) 80 may be external to the housing 101 of the IMD 100, yet still be implanted within or carried by the patient. Examples of physiologic sensors 80 might include sensors that, for example, sense respiration rate, pH of blood, ventricular gradient, activity, position/posture, minute ventilation (MV), and so forth. A particular example of a physiologic sensor is a non-optical PPG sensor. Such a PPG sensor is then provided to collect PPG signals as examples of biological signals.

A battery 90 provides operating power to all of the components in the IMD 100. The battery 90 is capable of operating at low current drains for long periods of time, and is capable of providing high-current pulses (for capacitor charging) when the patient requires a shock pulse, e.g., in excess of 2 A, at voltages above 2 V, for periods of 140 seconds or more. The battery 90 also desirably has a predictable discharge characteristic so that elective replacement time can be detected. As one example, the IMD 100 employs lithium/silver vanadium oxide batteries.

Optionally, the IMD 100 further includes an impedance measuring circuit (not shown), which can be used for many things, including lead impedance surveillance during the acute and chronic phases for proper lead positioning or dislodgement; detecting operable electrodes and automatically switching to an operable pair if dislodgement occurs; measuring respiration or minute ventilation; measuring thoracic impedance for determining shock thresholds; detecting when the device has been implanted; measuring stroke volume; and detecting the opening of heart valves; and so forth. The impedance measuring circuit is coupled to the electrode configuration switch 60 so that any desired electrode may be used.

Optionally, the IMD 100 also comprises a shocking circuitry 70 configured to generate shocking pulses of low (e.g., up to 0.5 joules), moderate (e.g., 0.5-10 joules), or high energy (e.g., 1311 to 40 joules), as controlled by the processor 30.

It is recognized that the configurations of circuitry and processors illustrated herein are by way of example only. Optionally, operations described in connection with the processor 30 may be implemented by circuitry, e.g., firmware and/or discrete circuitry. Optionally, operations described in connection with the circuitry, e.g., firmware and/or discrete circuitry, may be implemented by the processor 30.

In an embodiment, the sensing circuitry 40 is couplable, such as by the electrode configuration switch 60, to at least one electrode to thereby sense an electrical signal from a heart of the patient and generate a cardiac signal, also referred to as CA signal herein, based on the sensed electrical signal. Examples of the aforementioned electrodes are shown in and described above with reference to Figs. 1 and 2, but are not limited thereto. In such an embodiment, the processor 30 is configured to execute the specific executable instructions to normally analyze the cardiac or CA signal sensed by the sensing circuitry 40 to detect the COI indicative of the pathophysiologic condition of the patient. The processor 30 is also configured to denoise, when the IMD 100 is exposed to an EMI caused by a MR scanner, noisy cardiac or CA signal segments of the cardiac or CA signal sensed by the sensing circuitry 40 while the IMD 100 is exposed to the EMI using the denoising prediction model 400 to obtain denoised cardiac or CA signal segments. The processor 30 is further configured to analyze the denoised cardiac or CA signal segments to detect the COI indicative of the non-physiologic condition of the patient.

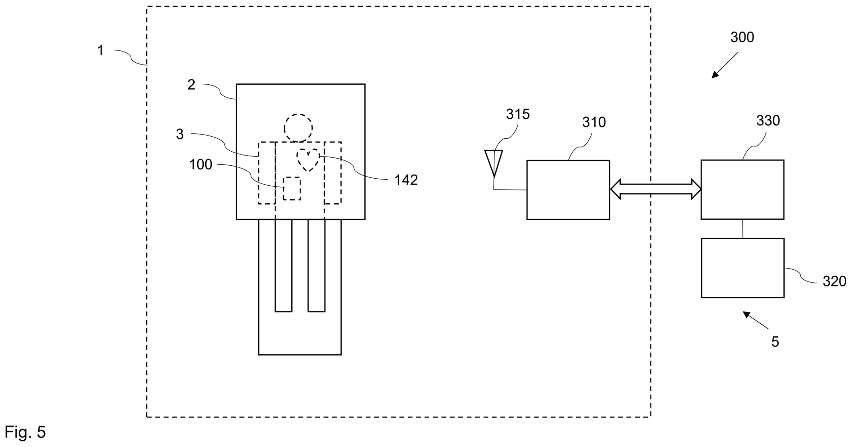

Hence, in an embodiment, the noise causing condition is exposure of EMI, such as MRI gradient induced currents. Fig. 5 illustrates an overall MRI system having an MR machine or scanner 2 operative to generate MRI fields during an MRI session for examining a patient 3. The MRI machine 2 operates under the control of an MR scanner console (not shown), which controls the strength and orientation of the fields generated by the MRI machine 2 and derives images of portions of the patient 3 therefrom, in accordance with otherwise conventional techniques. MRI machines 2 and imaging techniques are well known and will not be described in detail herein, see, for example, U.S. patent nos. 5,063,348 and 4,746,864. Optionally, an RF transceiver 310 connected to an antenna or communication coil 315 and a processor or controller 330 of an external device 300 are also provided to communicate during the MRI procedure with the IMD 100 implanted within the patient 3 to receive transmissions of noisy biological signals, or signal segments thereof, sensed within the patient 3 by the IMD 100 during the MRI session. The RF transceiver 310 may be omitted entirely if the IMD 100 performs the denoising on its own. The IMD 100 may be constructed in various manners.

The IMD 100 and/or lead system may also include various physiologic sensors for sensing hemodynamic signals or other signals within the patient, such as physiologic sensors operative to sense intracardiac pressure, blood oxygen saturation, e.g., SvO2, blood temperature, body temperature and PPG signals, etc. In any case, any of the various signals sensed by the physiologic sensor(s) can be used as biological signal as referred to herein.

Various configurations may be implemented to permit communication between the IMD 100 and the external device 300 during the MRI session. In the example of Fig. 5, the external device 300 comprises a communication circuitry 310 with connected antenna or communication coil 315 that communicates via medical implant communication system (MICS), Bluetooth^{®}, industrial, scientific and medical (ISM) channels and/or another wireless protocol, with corresponding communication components within the IMD 100. Wireless communication between the IMD 100 and the external device 300 is possible given that the MRI RF field (e.g. 64 MHz for 1.5 T, 128 MHZ for 3 T) and the gradient field (approximately 1 kHz) frequencies are well below the MICS and Bluetooth^{®} frequencies of 400 MHz and 2.4 GHz, respectively. The higher frequency harmonic signal caused by the MRI RF and gradient fields should be relatively minor as compared to wireless communication signal strength in the MICS and Bluetooth^{®} frequencies and should not affect wireless medication.

In an embodiment, the sensing circuitry 40 is couplable to an implantable lead 110, 112, 114 comprising at least one electrode 120, 123, 124, 126, 130, 132, 134, 136, 138 to thereby sense the electrical signal from the tissue.

In a particular embodiment, the noise causing condition is a lead failure of the implantable lead 110, 112, 114. Such a lead failure may cause the lead 110, 112, 114 connectable to the IMD 100 to capture various electrical signals produced by the patient body or external EMI as noise or interference on biological signals sensed by electrodes 120, 123, 124, 126, 130, 132, 134, 136, 138 and/or physiological sensors of the dysfunctional lead.

In an embodiment, the sensing circuitry 40 is couplable to a first intracardiac lead 112 comprising at least one electrode 120, 123 to thereby sense a first electric signal from a first cardiac chamber of a heart 142 and a second intracardiac lead 110 comprising at least one electrode 124, 126 to thereby sense a second electric signal from second cardiac chamber 140 of the heart 142 and configured to generate a first cardiac signal based on the first electric signal and a second cardiac signal based on the second electric signal. In such an embodiment, the processor 30 is configured to execute the specific executable instructions to denoise, when the IMD 100 is exposed to the noise causing condition, noisy cardiac signal segments of the first cardiac signal and of the second cardiac signal sensed by the sensing circuitry 40 while the IMD 100 is exposed to the noise causing condition using the denoising prediction model 400 to obtain the denoised cardiac signal segments. Thus, the embodiments of the present technology can be applied to the case of sensing multiple different biological signals, such as represented by electrical signals sensed at different cardiac chambers 140 of a heart 142 of the patient 3, such as in the right atrium, the right ventricle 140 and/or left ventricle.

In an alternatively embodiment, the sensing circuitry uses to generate the biological signal is a sensing circuitry of a sensor, such as the physiologic sensor 80 as illustrated in Fig. 3. The biological signal is then a sensor signal as generated by the physiologic sensor 80.

Illustrative, but non-limiting, examples of such physiologic sensors 80 include blood oxygen saturation sensor, temperature sensor, accelerometer, PPG sensor, pH sensor, heart sound sensor, impedance sensor, etc.

In the above-described embodiments, the denoising prediction model 400 and the optional segment classification model 450 are implemented locally in a memory 20 of the IMD 100 and thereby executed by the processor 30 of the IMD 100. As is further described herein, such an implementation is indeed feasible in terms of memory usage and computational complexity of the model(s) 400, 450. An alternative implementation is, though, to have the denoising prediction model 400 and the optional segment classification model 450 implemented remotely from the IMD 100, i.e., in an external device 300 capable of communicating wirelessly with the IMD 300, see Figs. 4 and 5.

Another aspect of the present technology therefore relates to a system 5 for denoising biological signals. The system 5 comprises an IMD 100 and an external device 300. The IMD 100 comprises a sensing circuitry 40 configured to generate a biological signal representative of a physiologic parameter of a patient 3. The IMD 100 also comprises a first communication circuitry 10 configured to perform wireless communication with the external device 300. The IMD 100 further comprises a first memory 20 storing specific executable instructions and a first processor 30 connected to the sensing circuitry 40, the first communication circuitry 10 and the first memory 20 and configured to execute the specific executable instructions (stored in the first memory 20) to normally analyze the biological signal sensed by the sensing circuitry 40 to detect a COI indicative of a pathophysiologic condition of the patient 3. The processor 30 is further configured to control, when the IMD 100 is exposed to a noise causing condition, the first communication circuitry 10 to transmit, to the external device 300, a biological signal generated by the sensing circuitry 40 while the IMD 100 is exposed to the noise causing condition, or biological signal segments thereof. The external device 300 comprises a second communication circuitry 310 configured to perform wireless communication with the IMD 100. The external device 300 also comprises a second memory 320 storing specific executable instructions and a denoising prediction model 400 trained to predict a denoised biological signal segment from a noisy biological signal segment. The external device 300 further comprises a second processor 330 connected to the second communication circuitry 320 and the second memory 320 and configured to execute the specific executable instructions (stored in the second memory 320) to denoise, using the denoising prediction model 400, noisy biological signal segments of the biological signal, or of the biological signal segments thereof, received from the second communication circuitry 310 from the IMD 100 to obtain denoised biological signal segments. The second processor 330 is also configured to control the second communication circuitry 310 to transmit, to the IMD 100, the denoised biological signal segments or information of the COI indicative of the pathophysiological condition of the patient 3 detected by analysis of the denoised biological signal segments. The specific executable instructions stored in the first memory 20 can also be referred to herein more specifically as the first specific executable instructions, and the specific executable instructions stored in the second memory 320 can also be referred to herein more specifically as the second specific executable instructions.

Reference to first and second communication circuitry, memory and processor herein is merely used to differentiate the communication circuitry 10, memory 20 or processor 30 of the IMD 100 from the communication circuitry 310, memory 320 or processor 330 of the external device 300. Thus, first communication circuitry 10, first memory 20 and first processor 30 as used herein indicate the communication circuitry 10, memory 20 and processor 30 of the IMD 100, whereas the second communication circuitry 310, second memory 320 and second processor 330 correspondingly indicate the communication circuitry 310, memory 320 and processor 330 of the external device 300.

In this embodiment, the IMD 100 senses biological signals using the sensing circuitry 40 of the patient 3 and normally, i.e., when the IMD 100 is not exposed to any noise causing condition, analyzes the biological signal generated by the sensing circuitry 40 to detect any pathophysiologic condition of the patient 3. However, when the IMD 100 is exposed to such a noise causing condition that thereby may introduce noise or interference in the biological signal generated by the sensing circuitry 40, the denoising of the noisy biological signal is performed by the external device 300, which, in this embodiment, has access to the denoising prediction model 400. Accordingly, the IMD 100 transmits the noisy biological signal, or signal segments thereof, to the external device 300 for denoising using the denoising prediction model 400. The denoised biological signal segments output by the denoising prediction model 400 can then be returned to the IMD 100 for analysis therein for the detection of the COI indicative of the pathophysiologic condition of the patient 3. Alternatively, the analysis of the denoised biological signal segments is performed by the external device 300, which then instead returns information of the COI indicative of the pathophysiological condition to the IMD 100.

Thus, in an embodiment, the second processor 330 is configured to execute the specific executable instructions to analyze the denoised biological signal segments to detect the COI indicative of the pathophysiologic condition of the patient 3 and control the second communication circuitry 310 to transmit the information of the COI indicative of the pathophysiologic condition of the patient 3 to the IMD 100. In this embodiment, the denoising and the analysis of the denoised biological signal segments are thereby performed by the external device 300, which thereby reduces the computations needed by the IMD 100 in order to detect the COI indicative of the pathophysiologic condition of the patient 3.

In another embodiment, the second processor 330 is configured to execute the specific executable instructions (stored in the second memory 320) to control the second communication circuitry 310 to transmit the denoised biological signal segments to the IMD 100. In this embodiment, the first processor 30 is then configured to execute the specific executable instructions (stored in the first memory 20) to analyze the denoised biological signal segments to detect the COI indicative of the pathophysiologic condition of the patient 3. In this embodiment, the denoising is performed by the external device 300 whereas the analysis of the denoised biological signal segments is performed by the first processor 30 of the IMD 100.

The various embodiments discussed in the foregoing of detecting or determining when the IMD 100 is exposed to the noise causing condition also applies to the system 5.

Thus, in an embodiment, the second communication circuitry 310 is configured to transmit a noise mode programming command to the IMD 100. In this embodiment, the first processor 30 is configured to execute the specific executable instructions (stored in the first memory 20) to control, in response to reception of the noise mode programming command, the first communication circuitry 10 to transmit, to the external device 300, the biological signal generated by the sensing circuitry 40 while the IMD 100 is exposed to the noise causing condition or the biological signal segments thereof.

In this embodiment, the external device 310 thereby transmits a noise mode programming command to the IMD 100, such as to instruct the IMD 100 to enter a noise operating mode. A typical example of such a situation is when a patient 3 comprising the IMD 100 is to undergo an MRI session as shown in Fig. 5. In such a case, prior to the start of the MRI session, the external device 300 can be used to program the IMD 100 to enter the noise operating mode by sending the noise programming command to the IMD 100. In such an embodiment, the second communication circuitry 310 is preferably arranged inside a shielded MRI room 1 together with the MR scanner 2, whereas the second processor 330 is preferably arranged outside of the shielded MRI room 1 but communicatively coupled to the second communication circuitry 310, preferably by a cable.

In an alternative, or additional, embodiment, the first processor 30 is configured to execute the specific executable instructions (stored in the first memory 20) to analyze the biological signal generated by the sensing circuitry 40 during a noise-sampling period to detect noise or interference in the biological signal. In such an embodiment, the first processor 30 is also configured to control, in response to detection of noise or interference in the biological signal, the first communication circuitry 10 to transmit, to the external device 300, the biological signal generated by the sensing circuitry 40 while the IMD 100 is exposed to the noise causing condition, or the biological signal segments thereof.

In this embodiment, the first processor 30 is preferably configured to execute a noise-detection algorithm that could be used to detect the presence of noise or interference on the biological signal as has been described in the foregoing.

In an alternative, or additional, embodiment, the IMD 100 comprises a communication coil 15 connected to the first communication circuitry 10. In this embodiment, the first processor 30 is configured to execute the specific executable instructions (stored in the first memory 20) to monitor, when the IMD 100 is exposed to noise causing condition, for noise on the communication coil 15 and control, when noise is detected on the communication coil 15, the first communication circuitry 10 to transmit, to the external device 300, the biological signal generated by the sensing circuitry 40 while the IMD 100 is exposed to the noise causing condition, or the biological signal segments thereof.

In this embodiment, the IMD 100 detects a noise causing condition by monitoring for noise or interference of the telemetry coil 15 of the first communication circuitry 10.

In an alternative, or additional, embodiment, the IMD 100 comprises a magnetic field sensor 85 configured to detect a magnetic field. In this embodiment, the first processor 30 is connected to the magnetic field sensor 85 and configured to execute the specific executable instructions (stored on the first memory 20) to control, in response to the magnetic field sensor 85 detecting the magnetic field, the first communication circuitry 10 to transmit, to the external device 300, the biological signal generated by the sensing circuitry 40 while the IMD 100 is exposed to the noise causing condition, or the biological signal segments thereof.

In this embodiment, the IMD 100 uses the magnetic field sensor 85 to detect a magnetic field or EMI to thereby determine when the IMD 100 is exposed to the noise causing condition.

The denoising prediction model 400 of the external device 300 uses (noisy) biological signal segments as input to predict corresponding denoised biological signal segments thereof. In an embodiment, the denoising prediction model 400 further uses an EMI-induced voltage as an additional input to predict the denoised biological signal segments as described in the foregoing.

In such an embodiment, the IMD 100 comprises a communication coil 15 connected to the first communication circuitry 10. The first processor 30 is, in this embodiment, configured to execute the specific executable instructions (stored in the first memory 20) to determine, when the IMD is exposed to EMI, an EMI-induced voltage on the communication coil 15, and control, when the IMD 100 is exposed to EMI, the first communication circuitry 10 to transmit, to the external device 300, the biological signal generated by the sensing circuitry 40 while the IMD 100 is exposed to the EMI, or the biological signal segments thereof, and the measured EMI-induced voltage on the communication coil 15. The second processor 330 is, in this embodiment, configured to execute the specific executable instructions (stored in the second memory 320) to denoise, using the denoising prediction model 400, the noisy biological signal segments to obtain the denoised biological signal segments by inputting the noisy biological signal segments and the measured EMI-induced voltage on the communication coil 15 into the denoising prediction model 400.

In an embodiment, the external device 300 not only stores the denoising prediction model 400 in the second memory 320 but also stores the segment classification model 450.

In such an embodiment, the second memory 320 stores a segment classification model 450 trained to classify biological signal segments as noisy or non-noisy biological signal segments. The second processor 330 is, in this embodiment, configured to execute the specific executable instructions (stored in the second memory 320) to classify the biological signal segments using the segment classification model 450. The second processor 330 is also configured to denoise, using the denoising prediction model 400, the biological signal segments classified as noisy by the segment classification model 450 to obtain the denoised biological signal segments.

Thus, in this embodiment, the external device 300 preferably only denoises those biological signal segments as received from the IMD 100, or derived from the biological signal received from the IMD 100, that the segment classification model 450 predicts as being noisy biological signal segments. Thus, those biological signal segments that are predicted to be non-noisy (i.e., those that are clean biological signal segments) are preferably not input into the denoising prediction model 400.

In an embodiment, the second processor 330 is configured to execute the specific executable instructions (stored in the second memory 320) to analyze the denoised biological signal segments and the biological signal segments classified as non-noisy by the segment classification model 450 to detect the COI indicative of the pathophysiologic condition of the patient 3.

Alternatively, the second processor 330 is configured to execute the specific executable instructions (stored in the second memory 320) to control the second communication circuitry 310 to transmit the denoised biological signal segments and the biological signal segments classified as non-noisy by the segment classification model 450 to the IMD 100. In such a case, the first processor 30, i.e., the processor 30 of the IMD 100, is configured to execute the specific executable instructions (stored in the first memory 20) to analyze the denoised biological signal segments and the biological signal segments classified as non-noisy by the segment classification model 450 to detect the COI indicative of the pathophysiologic condition of the patient 3.

In an embodiment, the segment classification model 450 at the external device 300 also uses an EMI-induced voltage on the communication coil 15 of the IMD 100 as input in addition to the biological signal segments. In such an embodiment, the IMD 100 comprises a communication coil 15 connected to the first communication circuitry 10. The first processor 30 is, in this embodiment, configured to execute the specific executable instructions (stored in the first memory 20) to determine, when the IMD 100 is exposed to EMI, an EMI-induced voltage on the communication coil 15. The first processor 30 is, in this embodiment, also configured to control, when the IMD 100 is exposed to EMI, the first communication circuitry 10 to transmit, to the external device 300, the biological signal generated by the sensing circuitry 40 while the IMD 100 is exposed to the EMI, or the biological signal segments thereof, and the determined EMI-induced voltage on the communication coil 15. The second processor 330 is, in this embodiment, configured execute the specific executable instructions (stored in the second memory 320) to classify the biological signal segments by inputting the biological signal segments and the measured EMI-induced voltage on the communication coil 15 into the segment classification model 450.

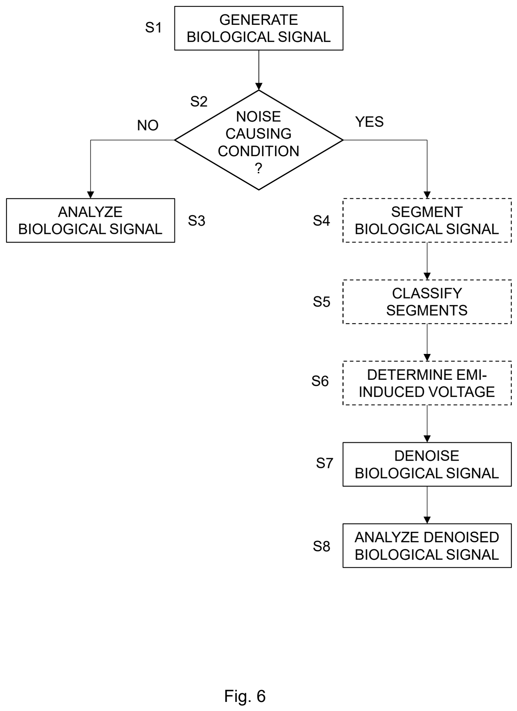

Fig. 6 is a flow chart illustrating a method for denoising biological signals according to an embodiment. The method comprises an IMD 100 generating, in step S1, a biological signal representative of a physiologic parameter of a patient 3. The IMD 100 normally analyzing, in step S3, the biological signal to detect a COI indicative of a pathophysiologic condition of the patient 3. The method also comprises denoising, in step S7 and when the IMD 100 is exposed to a noise causing condition, noisy biological signal segments of the biological signal generated while the IMD 100 is exposed to the noise causing condition using a denoising prediction model 400 to obtain denoised biological signal segments. The denoising prediction model 400 is trained to predict a denoised biological signal segment from a noisy biological signal segment. The method also comprises analyzing, in step S8, the denoised biological signal segments to detect the COI indicative of the pathophysiologic condition of the patient 3.

The analysis for the purpose of detecting any COI indicative of the pathophysiologic condition of the patient 3 is thereby performed in step S3 or in step S8 depending on whether the IMD 100 is exposed to a noise causing condition as determined in step S2. Thus, in normal conditions, i.e., when the IMD 100 is not exposed to any noise causing condition, the IMD 100 analyzes, in step S3, the biological signal generated in step S1. However, if the IMD 100 is exposed to a noise causing condition as determined in step S2, signal segments of the biological signal generated in step S1 are first denoised in step S7 prior to analysis of the denoised biological signal segments in step S8. Step S2 in Fig. 6 can be performed according to various embodiments.

In an embodiment, step S2 comprises reception of a noise mode programming command. In such a case, step S7 comprises denoising, in response to reception, in step S2, of the noise mode programming command, the noisy biological signal segments using the denoising prediction model 400 to obtain the denoised biological signal segments.

In an alternative, or additional, embodiment, the method further comprises analyzing, in step S2, the biological signal generated during a noise-sampling period to detect noise or interference in the biological signal. In such an embodiment, step S7 comprises denoising, in response to detection of noise or interference in the biological signal, the noisy biological signal segments using the denoising prediction model 400 to obtain the denoised biological signal segments.

In an alternative, or additional, embodiment, the method further comprises monitoring, in step S2 when the IMD 100 is exposed to the noise causing condition, for noise on a communication coil 15 of the IMD 100. In such an embodiment, step S7 comprises denoising, when noise is detected on the communication coil 15, the noisy biological signal segments using the denoising prediction model 400 to obtain the denoised biological signal segments.

In an alternative, or additional, embodiment, the method further comprises detecting, in step S2 and when the IMD 100 is exposed to the noise causing condition, a magnetic field. In such an embodiment, step S7 comprises denoising, when detecting the magnetic field, the noisy biological signal segments using the denoising prediction model 400 to obtain the denoised biological signal segments.

In an embodiment, the method comprises an additional, optional step S6. This step S6 comprises determining, when the IMD 100 is exposed to EMI, an EMI-induced voltage on a communication coil 15 of the IMD 100. In such an embodiment, step S7 comprises denoising, when the IMD 100 is exposed to EMI, the noisy biological signal segments to obtain the denoised biological signal segments by inputting the noisy biological signal segments and the determined EMI-induced voltage into the denoising prediction model 400.

In an embodiment, the method comprises additional, optional steps S4 and S5. Step S4 comprises segmenting, when the IMD 100 is exposed to the noise causing condition, the biological signal generated in step S1 while the IMD 100 is exposed to the noise causing condition into a plurality of biological signal segments. A next step S5 comprises classifying the plurality of biological signal segments using a segment classification model 450 trained to classify biological signal segments as noisy or non-noisy biological signal segments. In such an embodiment, step S7 comprises denoising the biological signal segments classified as noisy by the segment classification model 450 to obtain the denoised biological signal segments.

The segmentation of the biological signal in step S4 can be performed independent on step S5. Segmentation of the biological signal into biological signal segments can be performed according to various embodiments. For instance, the segmentation can involve dividing the biological signal into signal segments having a defined time duration, such as a duration selected within an interval of from 100 ms up to 1 s, such as 100 ms, 150 ms, 200 ms, 250 ms, 300 ms, 350 ms, 400 ms, 450 ms, 500 ms, 550 ms, 600 ms, 650 ms, 700 ms, 750 ms, 800 ms, 850 ms, 900 ms, 950 ms or 1000 ms as illustrative, but non-limiting, examples. Alternatively, the segmentation can involve dividing the biological signal into signal segments each comprising a defined number of signal samples, such as a number of signal samples selected within an interval of from 50 up to 500 signal samples, such as 50 samples, 60 samples, 70 samples, 80 samples, 90 samples, 100 samples, 125 samples, 150 samples, 175 samples, 200 samples, 225 samples, 250 samples, 275 samples, 300 samples, 325 samples, 350 samples, 375 samples, 400 samples, 425 samples, 450 samples, 475 samples, or 500 samples. Typically, the sensing circuitry 40 generates the biological signal with a given sampling rate, such as selected within an interval of from 100 up to 1000 Hz, such as from 250 up to 750 Hz. In such a case, having a defined number of signal samples is equivalent to having a defined time duration as the number of signal samples is equal to the sampling rate multiplied by the time duration.

In an embodiment, step S8 comprises analyzing the denoised biological signal segments and biological signal segments classified as non-noisy biological signal segments by the segment classification model 450 to detect the COI indicative of the pathophysiologic condition of the patient 3.

In an embodiment, the method further comprises step S6, which comprises determining, when the IMD 100 is exposed to EMI, an EMI-induced voltage on a communication coil 15 of the IMD 100. In such an embodiment, step S5 comprises classifying the plurality of biological signal segments by inputting the plurality of biological signal segments and the determined EMI-induced voltage on the communication coil 15 into the segment classification model 450.

In an embodiment, the method may additionally comprise deliver therapy in response to detection of the COI indicative of the pathophysiologic condition in the analysis performed in step S8. Thus, the processor 30 of the IMD 100 is preferably configured to executive the specific executable instructions to delivery therapy in response to detection of the COI indicative of the pathophysiologic condition.

As an example, the processor 30 could control the pulse generator 50 to generate a stimulation or pacing pulse for delivery by one or more electrodes coupled thereto. The processor 30 may, for instance, control the pulse generator 50 to deliver pacing pulses, anti-tachycardia pacing therapy, such as to treat fast rhythms (tachycardias), bradycardia pacing therapy, such as to treat slow heart rhythms (bradycardias), cardiac resynchronization therapy (CRT), such as to coordinate contractions of the left and right ventricles to improve the heart's efficiency, and the like depending on the particular pathophysiological condition, of which the detected COI is indicative.

Alternatively, or in addition, the processor 30 could control the shocking circuitry 70 generate shocking pulses, such as of low, moderate or high energy, for delivery by one or more electrodes coupled thereto. Illustrative, but non-limiting, examples of such shocking pulses include defibrillation shocks, such as to terminate ventricular fibrillation (VF), and cardioversion shocks, such as to treat ventricular tachycardia (VT), or atrial fibrillation (AF).

The methods described herein may employ structures or aspects of various embodiments (e.g., systems, devices and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all of such operations may be performed by the processor(s) of another device described herein.

The denoising prediction model 400 and the optional segment classification model 450 may be implemented according to various embodiments. For instance, the denoising prediction model 400 and/or the segment classification model 450 is a computer-implemented the denoising prediction model 400 and/or the segment classification model 450 and could be in the form a machine learning (ML) model. Generally, ML algorithms build a mathematical model based on training data, i.e., input biological signal segments and optional EMI-induced voltage according to the present technology, in order to make predictions or decisions without being explicitly programmed to do so. There are various types of ML algorithms that differ in their approach, the type of data they input and output, and the type of task or problem that they are intended to solve. Illustrative, but non-limiting, examples of such ML algorithms include supervised learning algorithms, unsupervised learning algorithms, semi-supervised learning algorithms, reinforcement learning algorithms, self-learning algorithms, feature learning algorithms, sparse dictionary learning algorithms, anomaly detection algorithms, and association rule learning algorithms.

Performing machine learning involves creating a model, which is trained on training data and can then process additional data to make predictions or decisions. Various types of ML models could be used according to the embodiments, including, but not limited to artificial neural networks, decision trees, support vector machines, regression analysis, Bayesian networks and Genetic algorithms.

Furthermore, deep learning, also known as deep structured learning, is a ML method based on artificial neural networks with representation learning. Learning can be supervised, semi-supervised or unsupervised. Deep learning architectures, such as deep neural networks, deep belief networks, recurrent neural networks and convolutional neural networks, could be used to train and implement the oxygen saturation estimation model. "Deep" in deep learning comes from the use of multiple layers in the network. Deep learning is concerned with an unbounded number of layers of bounded size, which permits practical application and optimized implementation, while retaining theoretical universality under mild conditions. In deep learning the layers are also permitted to be heterogeneous and to deviate widely from biologically informed connectionist models, for the sake of efficiency, trainability and understandability.

Particular examples of implementing the denoising prediction model (and the segment classification model) are in the form of a ML model comprising long-short term memory (LSTM) and one or multiple additional layers, such as selected from convolutional autoencoder layers, convolutional neural network layers, feedforward neural network layers, gated recurrent units layers, capsule networks layers, and radial basis function networks layers.

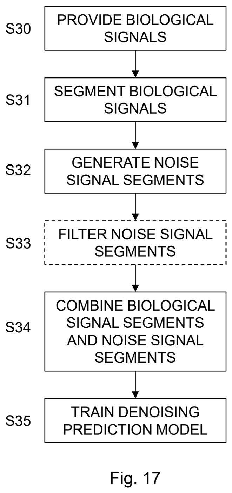

As an example, the denoising prediction model can be trained as shown in Fig. 17 providing a training data set to the model. In an embodiment, the training data set is provided by providing, in step S30, a plurality of biological signals sensed from a respective tissue of at least one patient, preferably from a plurality of patients. Each biological signal of the plurality of signals is segmented in step S31 into a plurality of biological signal segments. Noise or interference signal segments are generated in step S32, such as in the form of EMI signal segments or MRI-induced gradient noise signal segments. The noise or interference signal segments are preferably filtered in step S33, such as by a bandpass filter, to obtain filtered noise or interference signal segment. The plurality of biological signal segments and the filtered noise or interference signals are then combined in step S34 to obtain a plurality of noisy biological signal segments. In such a case, the training data set comprises the plurality of (clean) biological signal segments and the plurality of noisy biological signal segments. The denoising prediction model is then trained in step S35 based on the plurality of (clean) biological signal segments and the plurality of noisy biological signal segments.

Thus, the denoising prediction model is trained based on the training date set to predict, from an input noisy biological signal segment, a denoised biological signal segment that is as close as possible to the clean biological signal that was combined with a filtered noise or interference signal segment to obtain the noisy biological signal segment.

In an embodiment, the training data set also comprises the noise or interference signal segments as representative of an EMI-induced voltage signal segment on a telemetry coil 15 of the IMD 100.

The segment classification model can be trained in a similar manner to the denoising prediction model using a training data set comprising clean biological signal segments, noisy biological signals and information of whether a signal segment is clean or noisy. The training data set may optionally also comprise the noise or interference signal segments as representative of an EMI-induced voltage signal segment on a telemetry coil 15 of the IMD 100.

### IMPLEMENTATION EXAMPLE

In the following various implementation examples of the denoising prediction model 400 and the optional segment classification model 450 will be further described. These models 400, 450 are described with reference to biological signals in the form of CA signals sensed by electrodes couplable to an IMD. However, the embodiments of the present technology are, as described in the foregoing, not limited to such implementations. Furthermore, the noise causing condition is exemplified as EMI induced by an MR scanner. This should, however, merely be seen as an illustrative, but non-limiting, example of a noise causing condition.

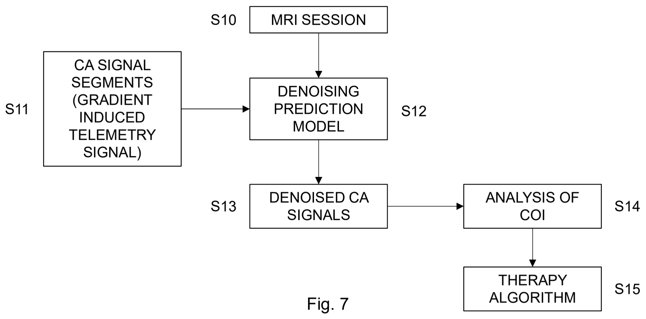

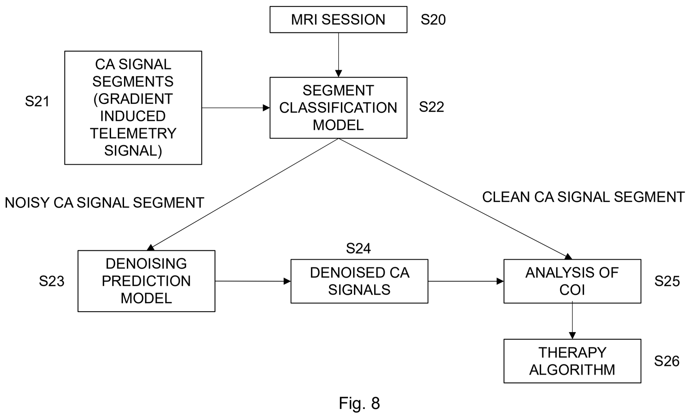

With reference to Fig. 7, if the IMD detects an MR scanner or is programmed into MRI mode during an MRI session in step S10, all CA signals sensed by the IMD in step S11 are treated as noisy cardiac signals, which are input into the denoising prediction model in step S11 and denoised by the denoising prediction model in step S13, prior to analysis of the IMD for the purpose of detection of any COI indicative of a pathophysiologic condition in step S14 that requires therapy in step S15. Correspondingly, in Fig. 8, if the IMD detects an MR scanner or is programmed into MRI mode during an MRI session in step S20, the CA signals sensed by the IMD in step S21 are first input into the segment classification model in step S22, which classifies that as noisy CA signal segments or clean CA signal segments. The noisy CA signal segments are input into the denoising prediction model in step S23 and denoised by the denoising prediction model in step S24, prior to analysis of the IMD for the purpose of detection of any COI indicative of a pathophysiologic condition in step S25 that requires therapy in step S25. The clean CA signal segments can, however, by used directly by the IMD for analysis for the purpose of detection of any COI indicative of the pathophysiologic condition in step S25.

The following models were trained:
- Single channel models - using CA signals, such as sensed by between RV ring and tip electrodes:
   ∘ segment classification model; and
   ∘ denoising prediction model;
- Dual channel models - using CA signals, such as sensed by between RV ring and tip electrodes, and gradient induced voltage on the communication coil;
   ∘ segment classification model; and
   ∘ denoising prediction model.

### Single channel - segment classification model

Matlab was used to train and evaluate a long short-term memory (LSTM) recurrent neural network deep learning model as segment classification model. The segment classification model used LSTM with the following parameter settings: MiniBatchsize: 18, Max Epochs: 50, Shuffle: Every Epoch, InitialLearnRate: 0.005 and Solver: Adam.

All signals used in the training and performance evaluation were divided into 250 ms long signal segments with 512 Hz sampling rate, implying 128 data points per signal segment. The segment classification model was trained with N=4920 clean cardiac signals from an EGM library.

The gradient noise signals (N=1901) were generated to reflect the waveform parameters presented in Table 1, which corresponds to Table 2 of ISO/TS 10974: 2018 Assessment of the safety of magnetic resonance imaging for patients with an active implantable medical device. Those waveform parameters were defined for safety assessment of active implantable medical device that sense during an MRI scan (TOFF max of 100 ms), with an induced gradient voltage up to 50 mV.

**Table 1 - Range of gradient test signal parameters**

| **Parameter** | **Values** | | | | | | | | | | | | |
|---|---|---|---|---|---|---|---|---|---|---|---|---|---|
| *B*_{G} (mT) | 1 | 2 | 5 | 10 | 30 | 40 | - | - | - | - | - | - | - |
| TSLEW (ms) | 0.3 | 0.4 | 1.0 | 2.0 | - | - | - | - | - | - | - | - | - |
| No. of cycles (burst length) | 1 | 2 | 5 | 10 | 20 | - | - | - | - | - | - | - | - |
| T_{OFF} (ms) | 0.2 | 0.5 | 1.0 | 2.0 | 5.0 | 10 | 20 | 50 | 100 | 200 | 500 | 1000 | 2000 |
| T_{DWELL} (ms) | 0.0 | 0.5 | 5.0 | 20 | - | - | - | - | - | - | - | - | - |
| Exposure time per sequence | 15 s minimum or characteristic response time of the device, if greater than 15 s | | | | | | | | | | | | |

One set of the gradient signals was filtered by the equivalent of a bandpass brady filter to represent the gradient noise signal as recorded by the sensing circuitry of the IMD.

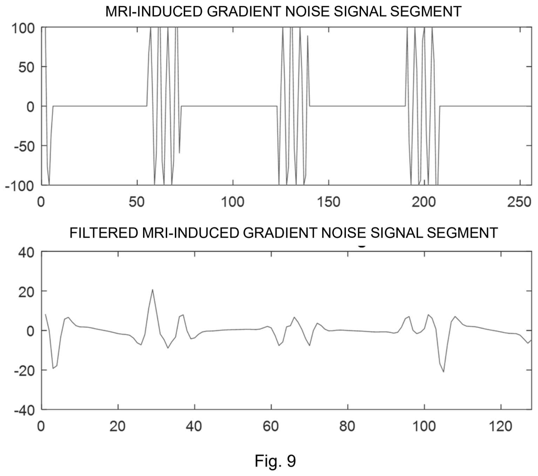

One set of the MRI-induced gradient noise signals represented the induced voltage on the communication coil and were therefore not filtered by the brady filter. An example of the unfiltered (before down sampling) and filtered MRI-induced noise gradient signal segments (after down sampling to 512 Hz) are shown in Fig. 9.

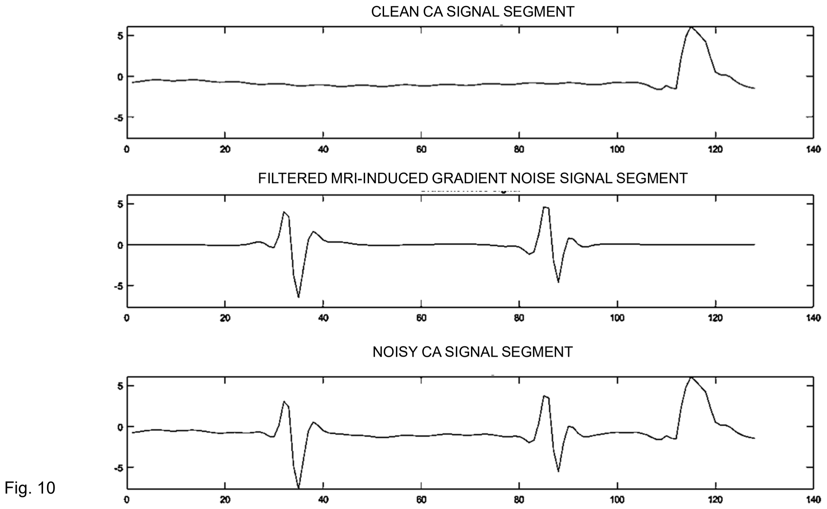

Noisy CA signals (N=1600) were generated by numerically adding the filtered gradient noise to the clean cardiac signals. An example of a CA signal segment (bottom) is shown in Fig. 10 along with the paired clean cardiac signal segment (top) and filtered gradient noise signal segment (middle).

The model accuracy was tested with (N=1230) clean CA signals from the EGM library, (N=400) noisy CA signals, and (N=475) filtered gradient noise signals. The performance results are shown in Table 2.

**Table 2 - performance of segment classification model**

| | **Predicted segment classification** | | | |
|---|---|---|---|---|
| **True segment classification** | | Gradient noise signal | Clean signal | Noisy signal |
| | Gradient noise signal | 471 | 1 | 12 |
| | Clean signal | 0 | 1220 | 53 |
| | Noisy signal | 4 | 9 | 335 |

The segment classification model had an accuracy of 96.3 % of classifying noisy CA signal segments (335 out of 348), 95.8 % accuracy of classifying clean CA signal segments (1220 out of 1273) and 97.3 % of classifying filtered gradient noise signal segments (471 out of 484) representing the induced voltage on the communication coil.

### Single channel - denoising prediction model

Matlab was used to train a deep learning model with bidirectional LSTM and convolutional autoencoder (ConvAE) layers with the following parameter settings: MiniBatchsize: 32, Max Epochs: 600, Shuffle: Every Epoch, InitialLearnRate: 0.001 and Solver: Adam.

The data pre-processing was the same as for the segment classification model described in the foregoing. The denoising prediction model was trained with (N=2000) clean CA signal segments from the EGM library and (N=2000) noisy CA signal segments, which were generated by numerically adding filtered gradient noise to the clean CA signal segments.

The ability of the denoising prediction model to accurately denoise noisy CA signal segments was evaluated using the following parameters:
1) Ratio: The ratio between the sum of squared value of (Denoised - Clean) and (Denoised - Noisy). This ratio indicated the similarity between the clean and denoised CA signal segments; a lower value indicated a stronger similarity. Denoised = denoised CA signal segment, Clean = clean CA signal segment, and Noisy = noisy CA signal segment.
2) Lag at Maximum Cross-Correlation: This value showed the lag (shift), at which the maximum similarity occurred.

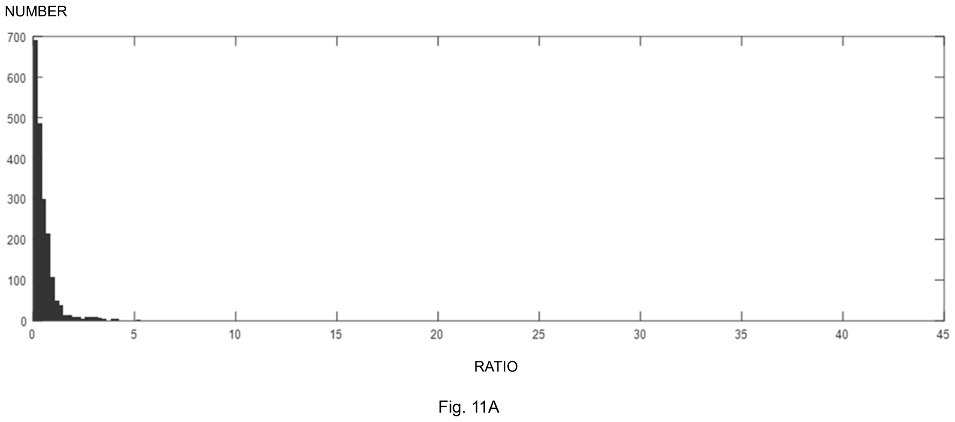

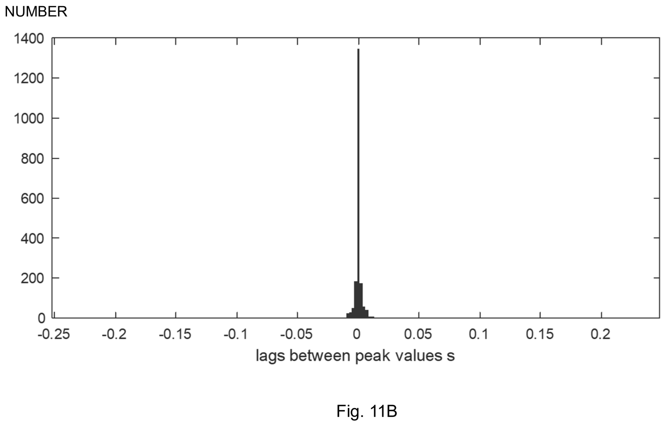

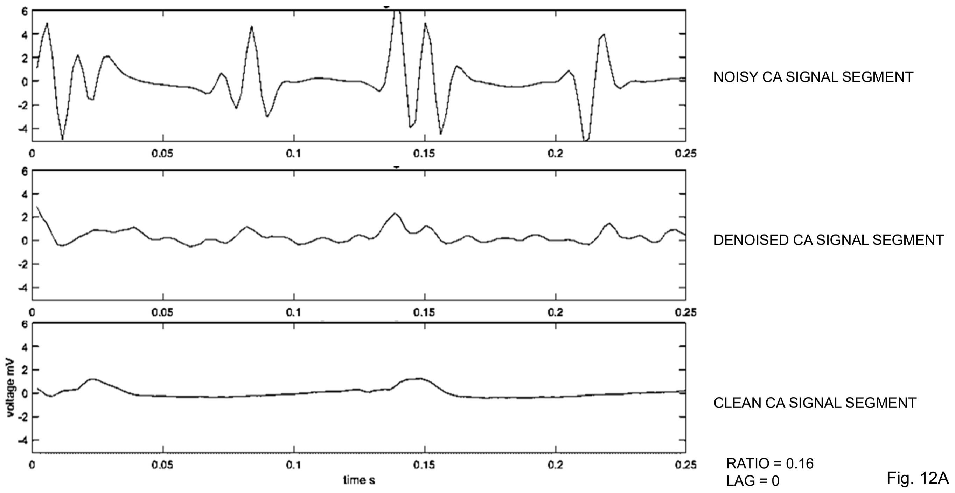

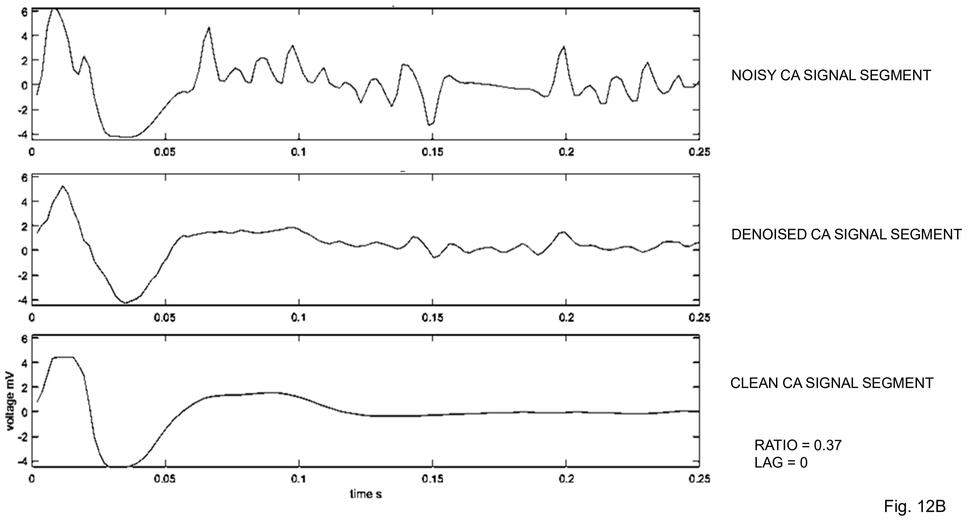

The results of the accuracy assessment are shown in Figs. 11A and 11B. These results showed that the majority (about 90%) of the denoised CA signal segments were more similar to clean CA signal segments, where the sum of squared value of (Denoised - Clean) was lower than the sum of squared value of (Denoised - Noisy), see Fig. 11A. The tight distribution of lag confirmed that the clean and denoised CA signal segments were aligned in time, i.e., they were paired data, see Fig. 11B. Figs. 12A and 12B illustrate two examples of noisy CA signal segment, denoised CA signal segment and clean CA signal segments (original) together with their ratio and lag at maximum cross-correlation values. The smaller the ratio value in Figs. 12A and 12B, the closer the denoised CA signal segment as predicted by the denoising prediction model is to the corresponding clean CA signal segment.

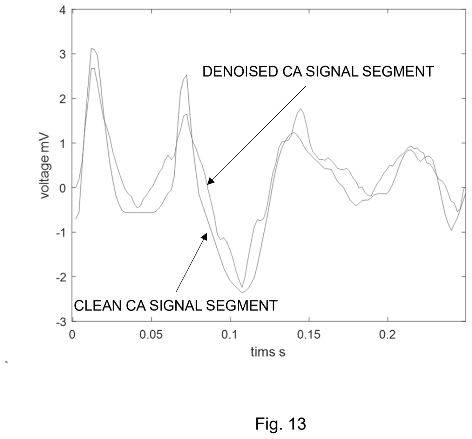

For implementation examples where classification of biological signal segments is not performed prior to prediction of denoised biological signal segments, there will be intermittent periods of time where there is no noise source and consequently no noise in the biological signal. Accordingly, non-noisy or clean biological signal segments will then be input into the denoising prediction model. Fig. 13 illustrates an example of such a non-noisy or clean CA signal segment and the denoised version thereof as output by the denoising prediction model if the non-noisy or clean CA signal segment is used as input.

### Dual channel - segment classification model

Matlab was used to train and evaluate a LSTM recurrent neural network deep learning model as segment classification model. The segment classification model used LSTM with the following parameter settings: MiniBatchsize: 18, Max Epochs: 18, Shuffle: Every Epoch, InitialLearnRate: 0.005 and Solver: Adam.

The pre-processing of data and the training set were the same as described in the single channel segment classification model, except paired cardiac and unfiltered gradient data was used, (N=4920) pairs of clean CA signal segments and zero gradient signal segments, and (N=1600) pairs of noisy CA signal segments + unfiltered gradient signal segments) to generate dual channel data.

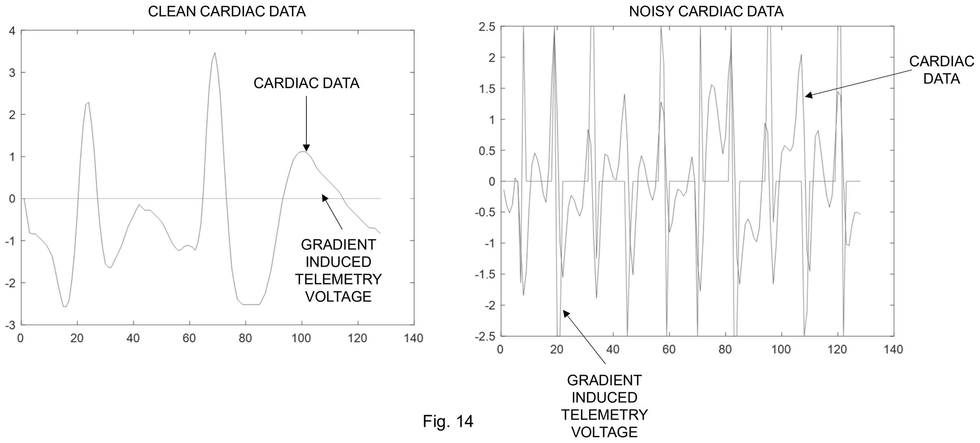

Fig. 14 illustrates an example of paired clean cardiac and noisy cardiac training data.

Performance assessment of the segment classification model used (N=1230) signal pairs for clean cardiac data and (N=400) signal pairs for noisy cardiac data. The results are presented in Table 3.

**Table 3 - performance of segment classification model**

| | | **Predicted segment classification** | |
|---|---|---|---|
| | | Clean signal | Noisy signal |
| **True segment classificatio**n | Clean signal | 1230 | 17 |
| | Noisy signal | 0 | 383 |

The segment classification model had an accuracy of 100 % of classifying noisy CA signal segments (383 out of 383) and an accuracy of 97.5 % of classifying clean CA signal segments (1230 out of 1247).

### Dual channel - denoising prediction model

Matlab was used to train a deep learning model with bidirectional LSTM and convolutional autoencoder (ConvAE) layers with the following parameter settings: MiniBatchsize: 32, Max Epochs: 600, Shuffle: Every Epoch, InitialLearnRate: 0.001 and Solver: Adam.

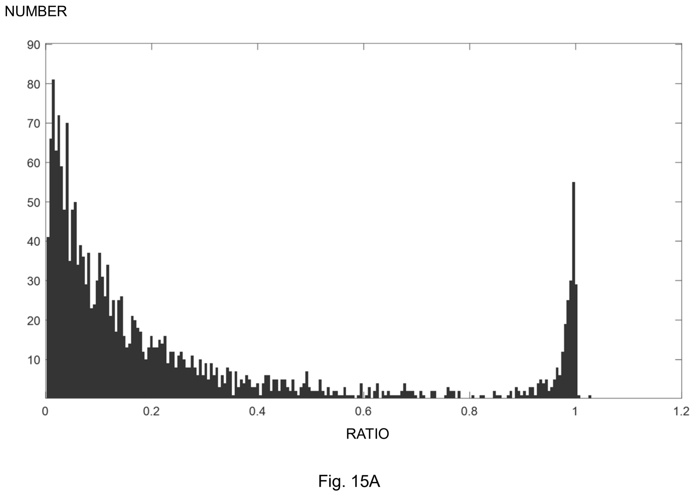

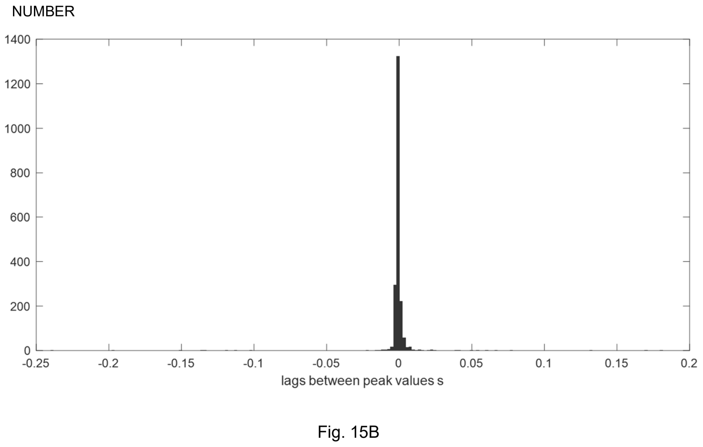

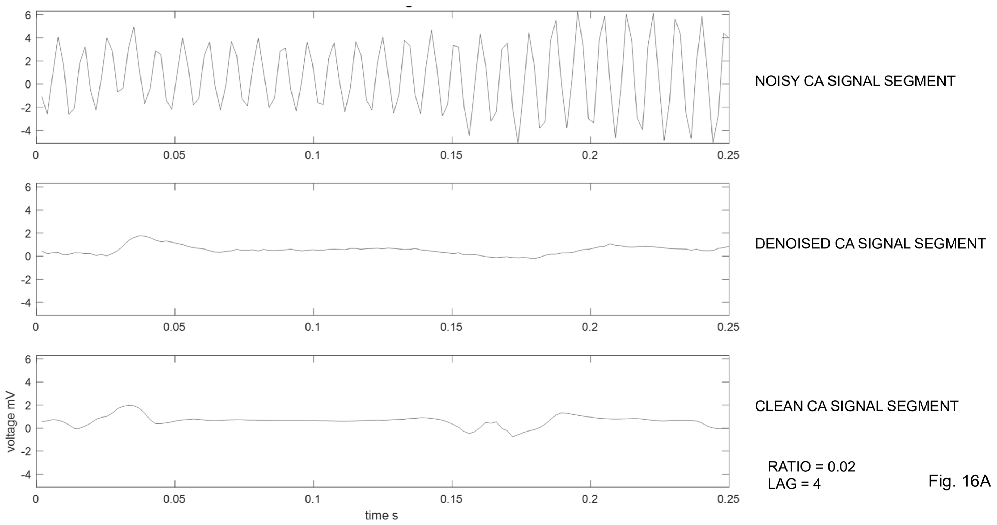

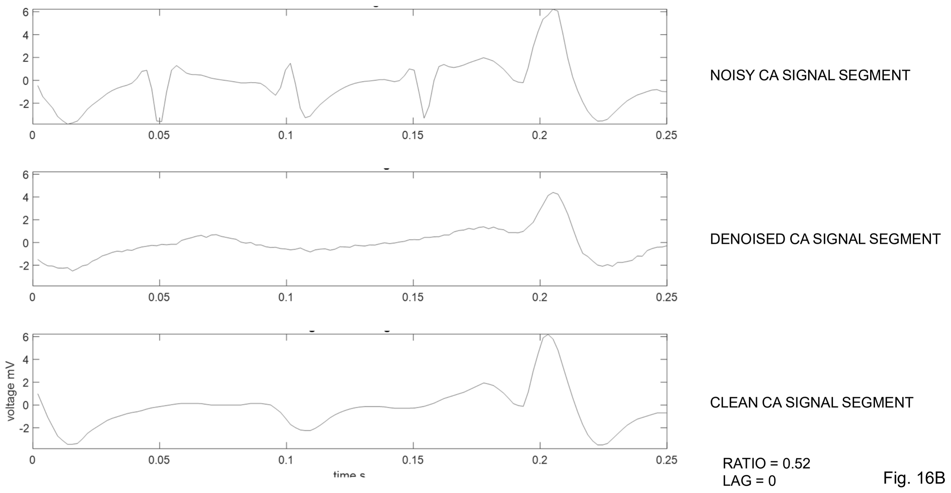

The pre-processing of data and the training set were the same as described in the dual channel denoising prediction model. The performance assessment used (N=2000) pairs of noisy CA signal segments and unfiltered gradient signal segments. The ratio and lags between peak values (Figs. 15A and 15B) were calculated with an improvement seen compared to the single channel denoising prediction model, as shown by lower ratio upper bound in Fig. 15A. Figs. 16A and 16B illustrate two examples of noisy CA signal segment, denoised CA signal segment and clean CA signal segments (original) together with their ratio and lag at maximum cross-correlation values.

The dual channel denoising prediction model with the incorporation of the gradient induced voltage on the telemetry coil improved the predictability of the denoised CA signal segments compared to the single channel denoising predication model.

### Hardware implementation

The model parameters are shown in Table 4 below without any model compression.

**Table 4 - Model parameter memory estimates**

| **Model** | **Parameter memory (kB)** | **Number of operations** | **Number of Learnables** |
|---|---|---|---|
| Single channel - segment classification model | 465 | 124,200 | 121,503 |
| Single channel - denoising prediction model | 4.8 | 3,336 | 1,265 |
| Dual channel - segment classification model | 465 | 124,612 | 121,911 |
| Dual channel - denoising prediction model | 4.8 | 3,352 | 1,275 |

The Matlab code can be ported to C code for incorporation into an embedded system. A microcontroller (MCU) used widely for embedded deep learning applications is the ARM Cortex-M series (Orasan et al., A brief review of deep neural network implementation for ARM Cortex-M processor, Electronics (2022) 11(16): 2545). Assuming the operations can be performed in fixed point, a rough estimate of the latency for the dual channel denoising model is 33 ms based on the parameters in Table 4, 20 cycles per operation, 2 MHz clock and Cortex M23. The implementation is estimated to use 4.8 kB of RAM and 67 kcycles (= 3336 operations * 20 cycles/operation).

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, it is noted that the term "based on" as used herein, unless stated otherwise, should be interpreted as meaning based at least in part on, meaning there can be one or more additional factors upon which a decision or the like is made. For example, if a decision is based on the results of a comparison, that decision can also be based on one or more other factors in addition to being based on results of the comparison.

It is to be understood that the above description is intended to be illustrative, and not restrictive. For example, the above-described embodiments (and/or aspects thereof) may be used in combination with each other. In addition, many modifications may be made to adapt a particular situation or material to the teachings of the embodiments of the present technology without departing from its scope. While the dimensions, types of materials and coatings described herein are intended to define the parameters of the embodiments of the present technology, they are by no means limiting and are exemplary embodiments. Many other embodiments will be apparent to those of skill in the art upon reviewing the above description. The scope of the embodiments of the present technology should, therefore, be determined with reference to the appended claims, along with the full scope of equivalents to which such claims are entitled. In the appended claims, the terms "including" and "in which" are used as the plain-English equivalents of the respective terms "comprising" and "wherein." Moreover, the terms "first," "second," and "third," etc. are used merely as labels, and are not intended to impose numerical requirements on their objects.

## Claims

1. An implantable medical device, IMD, (100) comprising:
a sensing circuitry (40) configured to generate a biological signal representative of a physiologic parameter of a patient (3);
a memory (20) storing specific executable instructions and a denoising prediction model (400) trained to predict a denoised biological signal segment from a noisy biological signal segment; and
a processor (30) connected to the sensing circuitry (40) and the memory (20) and configured to execute the specific executable instructions to:
normally analyze the biological signal generated by the sensing circuitry to detect a characteristic of interest, COI, indicative of a pathophysiologic condition of the patient (3);
denoise, when the IMD (100) is exposed to a noise causing condition, noisy biological signal segments of the biological signal generated by the sensing circuitry while the IMD (100) is exposed to the noise causing condition using the denoising prediction model (400) to obtain denoised biological signal segments; and
analyze the denoised biological signal segments to detect the COI indicative of the pathophysiologic condition of the patient (3).

2. The IMD of claim 1, further comprising a communication circuitry (10) configured to receive a noise mode programming command from an external device (300), wherein the processor (30) is connected to the communication circuitry (10) and is configured to execute the specific executable instructions to denoise, in response to reception of the noise mode programming command, the noisy biological signal segments using the denoising prediction model (400) to obtain the denoised biological signal segments.

3. The IMD of claim 1 or 2, wherein the processor (30) is configured to execute the specific executable instructions to:
analyze the biological signal generated by the sensing circuitry (40) during a noise-sampling period to detect noise or interference in the biological signal; and
denoise, in response to detection of the noise or interference in the biological signal, the noisy biological signal segments using the denoising prediction model to obtain the denoised biological signal segments.

4. The IMD of any one of claims 1 to 3, further comprising a communication circuitry (10) connected to a communication coil (15), wherein the processor (30) is connected to the communication circuitry (10) and is configured to execute the specific executable instructions to:
monitor for noise on the communication coil (15) to thereby detect when the IMD (100) is exposed to the noise causing condition; and
denoise, when noise is detected on the communication coil (15), the noisy biological signal segments using the denoising prediction model (400) to obtain the denoised biological signal segments.

5. The IMD of any one of claims 1 to 4, further comprising a magnetic field sensor (85) configured to detect a magnetic field, wherein the processor (30) is connected to the magnetic field sensor (85) and is configured to execute the specific executable instructions to:
denoise, in response to the magnetic field sensor (85) detecting the magnetic field, the noisy biological signal segments using the denoising prediction model (400) to obtain the denoised biological signal segments.

6. The IMD of any one of claims 1 to 5, further comprising a communication circuitry (10) connected to a communication coil (15), wherein the processor (30) is connected to the communication circuitry (10) and is configured to execute the specific executable instructions to:
determine, when the IMD is exposed to electromagnetic interference, EMI, an EMI-induced voltage on the communication coil (15); and
denoise, when the IMD (100) is exposed to EMI, the noisy biological signal segments to obtain the denoised biological signal segments by inputting the noisy biological signal segments and the determined EMI-induced voltage on the communication coil (15) into the denoising prediction model (400).

7. The IMD of any one of claims 1 to 6, wherein:
the memory (20) stores a segment classification model (450) trained to classify biological signal segments as noisy or non-noisy biological signal segments; and
the processor (30) is configured to execute the specific executable instructions to:
segment, when the IMD (100) is exposed to the noise causing condition, the biological signal generated by the sensing circuitry (40) while the IMD (100) is exposed to the noise causing condition into a plurality of biological signal segments;
classify the plurality of biological signal segments using the segment classification model (450); and
denoise the biological signal segments classified as noisy by the segment classification model (450) using the denoising prediction model (400) to obtain denoised biological signal segments; and optionally analyze the biological signal segments classified as non-noisy by the segment classification model (450), without any denoising of the biological signal segments classified as non-noisy, to detect the COI indicative of the pathophysiologic condition of the patient (3).

8. The IMD of claim 7, further comprising a communication circuitry (10) connected to a communication coil (15), wherein the processor (30) is connected to the communication circuitry (10) and is configured to execute the specific executable instructions to:
determine, when the IMD (100) is exposed to electromagnetic interference, EMI, an EMI-induced voltage on the communication coil (15); and
classify the plurality of biological signal segments by inputting the plurality of biological signal segments and the determined EMI-induced voltage on the communication coil (15) into the segment classification model (450).

9. The IMD of any one of claims 1 to 6, further comprising a communication circuitry (10) configured to:
transmit, to an external device (300) comprising a segment classification model (450) trained to classify biological signal segments as noisy or non-noisy biological signal segments, the biological signal generated by the sensing circuitry (40) while the IMD (100) is exposed to the noise causing condition or a plurality of biological signal segments thereof; and
receive, from the external device (300), a respective classification of the plurality of biological signal segments as noisy or non-noisy, wherein the processor (30) is connected to the communication circuitry (10) and is configured to execute the specific executable instructions to denoise biological signal segments classified as noisy by the segment classification model (450) using the denoising prediction model (400) to obtain denoised biological signal segments.

10. The IMD of claim 9, further comprising a communication coil (15) connected to the communication circuitry (10), wherein:
the processor (30) is configured to execute the specific executable instructions to determine, when the IMD (100) is exposed to electromagnetic interference, EMI, an EMI-induced voltage on the communication coil (15); and
the communication circuitry (10) is configured to transmit, to the external device (300), the biological signal sensed by the sensing circuitry (40) while the IMD (100) is exposed to the EMI, or the plurality of biological signal segments thereof, and the determined EMI-induced voltage on the communication coil (15).

11. The IMD of any one of claims 1 to 10, wherein:
the sensing circuitry (40) is couplable to at least one electrode (116, 120, 122, 123, 124, 126, 129, 130, 132, 134, 136, 138, 220, 225) to thereby sense an electrical signal from a tissue (142) and configured to generate the biological signal based on the sensed electrical signal; or
the IMD (100) further comprising a sensor (80) comprising the sensing circuitry.

12. The IMD of claim 11, wherein:
the sensing circuitry (40) is couplable to the at least one electrode (116, 120, 122, 123, 124, 126, 129, 130, 132, 134, 136, 138, 220, 225) to thereby sense an electrical signal from a heart (142) of the patient (3) and generate a cardiac signal based on the sensed electrical signal; and
the processor (30) is configured to execute the specific executable instructions to:
normally analyze the cardiac signal sensed by the sensing circuitry (40) to detect the COI indicative of the pathophysiologic condition of the patient (3);
denoise, when the IMD (100) is exposed to an electromagnetic interference, EMI, caused by a magnetic resonance imaging, MRI scanner (2), noisy cardiac signal segments of the cardiac signal sensed by the sensing circuitry (40) while the IMD (100) is exposed to the EMI using the denoising prediction model (400) to obtain denoised cardiac signal segments; and
analyze the denoised cardiac signal segments to detect the COI indicative of the pathophysiologic condition of the patient (3).

13. The IMD of any one of claims 1 to 12, wherein:
the sensing circuitry (40) is couplable to an implantable lead (110, 112, 114) comprising at least one electrode (116, 120, 122, 123, 124, 126, 129, 130, 132, 134, 136, 138) to thereby sense the electrical signal from a tissue (142) and the noise causing condition is a lead failure of the implantable lead (110, 112, 114); and/or
the noise causing condition is exposure to electromagnetic interference, EMI, preferably magnetic resonance imaging, MRI, gradient induced currents.

14. A system (5) for denoising biological signals comprising:
an implantable medical device, IMD, (100) comprising:
a sensing circuitry (40) configured to generate a biological signal representative of a physiologic parameter of a patient (3);
a first communication circuitry (10) configured to perform wireless communication with an external device (300);
a first memory (20) storing first specific executable instructions; and
a first processor (30) connected to the sensing circuitry (40), the first communication circuitry (10) and the first memory (20) and configured to execute the first specific executable instructions to:
normally analyze the biological signal sensed by the sensing circuitry (40) to detect a characteristic of interest, COI, indicative of a pathophysiologic condition of the patient (3);
control, when the IMD (100) is exposed to a noise causing condition, the first communication circuitry (10) to transmit, to the external device (300), a biological signal generated by the sensing circuitry (40) while the IMD (100) is exposed to the noise causing condition, or biological signal segments thereof; and
the external device (300) comprising:
a second communication circuitry (310) configured to perform wireless communication with the IMD (100);
a second memory (320) storing second specific executable instructions and a denoising prediction model (400) trained to predict a denoised biological signal segment from a noisy biological signal segment; and
a second processor (330) connected to the second communication circuitry (310) and the second memory (320) and configured to execute the second specific executable instructions to:
denoise, using the denoising prediction model (400), noisy biological signal segments of the biological signal, or of the biological signal segments thereof, received by the second communication circuitry (310) from the IMD (100) to obtain denoised biological signal segments; and
control the second communication circuitry (310) to transmit, to the IMD (100), the denoised biological signal segments or information of the COI indicative of the pathophysiologic condition of the patient (3) detected by analysis of the denoised biological signal segments.

15. The system of claim 14, wherein:
the second processor (330) is configured to execute the second specific executable instructions to analyze the denoised biological signal segments to detect the COI indicative of the pathophysiologic condition of the patient (3), and to control the second communication circuitry (310) to transmit the information of the COI indicative of the pathophysiologic condition of the patient (3) to the IMD (100); or
the second processor (330) is configured to execute the second specific executable instructions to control the second communication circuitry (310) to transmit the denoised biological signal segments to the IMD (100), and the first processor (30) is configured to execute the first specific executable instructions to analyze the denoised biological signal segments to detect the COI indicative of the pathophysiologic condition of the patient (3).
